# EUROPÄISCHE PATENTSCHRIFT

(11) **EP 0 702 013 B1**
(45) Veröffentlichungstag und Bekanntmachung des Hinweises auf die Patenterteilung: **13.06.2001**
(21) Anmeldenummer: 95113840.3
(22) Anmeldetag: 04.09.1995
(51) Int. Cl.: C07D 471/04, A61K 31/435

(54) **Imidazopyridine**
Imidazopyridines
Imidazopyridines

(30) Priorität: 15.09.1994 DE 4432860
(43) Veröffentlichungstag der Anmeldung: 20.03.1996
(73) Patentinhaber: MERCK PATENT GmbH, 64293 Darmstadt (DE)
(72) Erfinder: Mederski, Werner, Dr., D-64390 Erzhausen (DE); Osswald, Mathias, Dr., D-64673 Zwingenberg (DE); Schelling, Pierre, Prof. Dr., D-64367 Mühltal (DE); Minck, Klaus-Otto, Dr., D-64372 Ober-Ramstadt (DE); Beier, Norbert, Dr., D-64354 Reinheim (DE); Lues, Ingeborg, Dr., D-64297 Darmstadt (DE); Dorsch, Dieter, Dr., D-64372 Ober-Ramstadt (DE)

(56) Entgegenhaltungen:
- EP-A- 0 400 974
- EP-A- 0 505 893

## Beschreibung

Die Erfindung betrifft neue Imidazopyridinderivate der Formel I 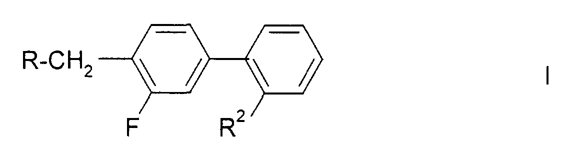 worin
- R: 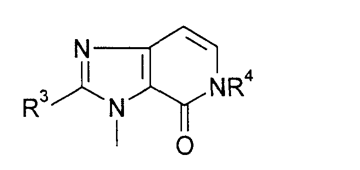
- R²: -SO₂NH-COR⁵,
- R³: A, C₂-C₆-Alkenyl, C₂-C₆-Alkinyl, C₃-C₈-Cycloalkyl-CₖH₂ₖ- oder C₁-C₆-Alkyl, worin eine CH₂-Gruppe durch O oder S ersetzt ist,
- R⁴: R⁸, unsubstituiertes oder ein- oder mehrfach durch COOH, COOA, CN, NO₂, NR⁶R⁷, NHCOR⁸, NHSO₂R⁸, Hal und/oder Ar substituiertes C₂-C₆-Alkenyl, C₂-C₆-Alkinyl, -CₙH₂ₙ-R⁹ oder-CHR¹⁰-CₖH₂ₖ-R¹¹,
- R⁵: A, -CₜH₂ₜ-(C₃-C₈-Cycloalkyl), -CₜH₂ₜ-Ar, -OA, -O-CₜH₂ₜ-(C₃-C₈-Cycloalkyl), -O-CₜH₂ₜ-Ar, -CₚH₂ₚ-O-C₃-C₈-Cycloalkyl oder -CₚH₂ₚ-O-Ar,
- R⁶ und R⁷: jeweils H, A, C₂-C₆-Alkenyl oder C₂-C₆-Alkinyl, Ar, ArCₙH₂ₙ- oder Het²,
- R⁶: auch -CH₂COOA, -SO₂-A oder -SO₂-Ar,
- R⁶ und R⁷: zusammen auch eine Alkylenkette mit 2-5 C-Atomen, die ein-oder mehrfach durch Carbonylsauerstoff, Ar, Het², -CO-Ar, - COOA, -CO-N(A)₂, -CH₂OH, -SO₂-Ar und/oder -NH-CO-A substituiert und/oder durch O oder durch -NR¹⁶- unterbrochen sein kann,
- R⁸: C₁-C₅-Alkyl, worin auch ein oder mehrere H-Atom(e) durch F ersetzt sein können,
- R⁹: C₃-C₈-Cycloalkyl, CN, COOA, COOH, Ar, Het¹, Het², 1H-5-Tetrazolyl, -CO-NR⁶R⁷, -CO-R⁸, -CO-Ar, -CO-Het², -CO-R¹⁴,-C(=NR¹²)-A, -C(=NR¹²)-Het², -S(O)ₘ-A, -S(O)ₘ-Ar, -S(O)ₘ-Het², -SO₂-NH-Het² oder -SO₂-OR¹⁵,
- R¹⁰: COOH, COOA, CONR⁶R⁷, CN, NO₂, NHCOR¹¹, NHSO₂R¹¹ oder 1H-5-Tetrazolyl,
- R¹¹: Ar oder Cycloalkyl mit 3-8 C-Atomen,
- R¹²: H, OH, CN, R¹³, OR¹³ oder OAr,
- R¹³: A, C₂-C₆-Alkenyl oder C₂-C₆-Alkinyl,
- R¹⁴: -NH-CHR¹⁵-COOH, -NH-CHR¹⁵-COOA, -CH₂S(O)ₘ-Ar, -CH₂-COOA, -CₙH₂ₙ-NO₂, -CₙH₂ₙ-NR⁶R⁷ oder -CₙH₂ₙ-NHCOOA,
- R¹⁵: H oder A,
- R¹⁶: H, A, Ar, COOA, Het² oder SO₂-Ar,
- A: C₁-C₆-Alkyl,
- Ar: eine unsubstituierte oder eine durch R⁸, OH, OR¹¹, COOH, COOA, CONH₂, CONHA, CON(A)₂, CH₂OH, CH₂OA, CN, NO₂, NH₂, NHA, N(A)₂, NHCOR¹¹, NHCOOA, NHSO₂R⁸, Hal und/oder 1 H-5-Tetrazolyl mono- oder disubstituierte Phenylgruppe,
- Het¹: einen fünf- oder sechsgliedrigen gesättigten heterocyclischen Rest mit 1 bis 3 N-, O- und/oder S-Atomen, der einfach durch Carbonylsauerstoff oder =NR¹² und/oder dessen Ring-N-Atom(e) jeweils durch A oder Ar substituiert sein kann (können),
- Het²: einen fünf- oder sechsgliedrigen heteroaromatischen Rest mit 1 bis 3 N-, O- und/oder S-Atomen, der auch mit einem Benzol-oder Pyridinring kondensiert sein kann,
- Hal: F, Cl, Br oder I,
- k und t: jeweils 0, 1, 2, 3 oder 4,
- m: 0, 1 oder 2,
- n: 1, 2, 3, 4, 5 oder 6 und
- p: 1 oder 2 bedeuten, sowie ihre Salze.

Ähnliche Verbindungen sind aus der EP-A2-0400 974 bekannt. Andere Imidazopyridinderivate mit angiotensin II-antagonistischen Eigenschaften und AT₁-Selektivität sind aus der EP 505 893 bekannt.

Der Erfindung lag die Aufgabe zugrunde, neue Verbindungen mit wertvollen Eigenschaften aufzufinden, insbesondere solche, die zur Herstellung von Arzneimitteln verwendet werden können.

Es wurde gefunden, daß die Verbindungen der Formel I und ihre Salze bei guter Verträglichkeit sehr wertvolle pharmakologische Eigenschaften besitzen. Insbesondere zeigen sie angiotensin II-antagonistische Eigenschaften und können daher als Arzneimittelwirkstoffe zur Prophylaxe und/oder Therapie von Herz-, Kreislauf- und Gefäßkrankheiten, vor allem zur Behandlung der angiotensin II-abhängigen Hypertension, des Aldosteronismus, der Herzinsuffizienz und des erhöhten Augeninnendrucks sowie von Störungen des Zentralnervensystems eingesetzt werden, ferner von Hypertrophie und Hyperplasie der Blutgefäße und des Herzens, Angina pectoris, Herzinfarkt, Schlaganfall, Restenosen nach Angioplastie oder By-pass-Operationen, Arteriosklerose, Glaukomen, macularer Degeneration, Hyperurikämie, Nierenfunktionsstörungen, z.B. Nierenversagen, Nephropathia diabetica, Retinopathia diabetica, Psoriasis, angiotensin II-vermittelten Störungen in weiblichen Fortpflanzungsorganen, Wahrnehmungsstörungen, z.B. Demenz, Amnesie, Gedächtnisfunktionsstörungen, Angstzuständen, Depression und/oder Epilepsie.

Diese Wirkungen können nach üblichen in vitro- oder in vivo-Methoden ermittelt werden, wie sie z.B. in der US-PS 4 880 804, der US-PS 5 036 048 und der WO 91/14367 beschrieben sind, ferner von A.T. Chiu et al., J. Pharmacol. Exp. Therap. 250, 867-874 (1989), und von P.C. Wong et al., ibid. 252, 719-725 (1990; in vivo, an Ratten).

Insbesondere haben diese Verbindungen eine hohe Affinität zum AT₁- und zum AT₂-Rezeptor, feststellbar z.B. an der Nebennierenmedulla von Ratten nach S. Whitebread et al., Biochem. Biophys. Res. Commun. 163, 284-291 (1989) sowie nach A.T. Chiu et al., Eur. J. Pharmacol. 170, 117-118 (1989). Außerdem weisen die Verbindungen einen funktionellen Antagonismus am AT₁-Rezeptor auf.

Gegenstand der Erfindung sind die Verbindungen der Formel I und ihre Salze sowie ein Verfahren zur Herstellung dieser Verbindungen sowie ihrer Salze, dadurch gekennzeichnet, daß man
(a) eine Verbindung, die der Formel I entspricht, aber an Stelle des Restes R² eine -SO₂NH₂-Gruppe trägt, mit einer Verbindung der Formel E-COR⁵ umsetzt oder
(b) eine Verbindung der Formel I aus einem ihrer funktionellen Derivate durch Behandeln mit einem solvolysierenden oder hydrogenolysierenden Mittel in Freiheit setzt,
und/oder daß man in einer Verbindung der Formel I einen oder mehrere Rest(e) R und/oder R in einen oder mehrere andere Reste R und/oder R² umwandelt und/oder eine Base oder Säure der Formel I in eines ihrer Salze umwandelt.

Vor- und nachstehend haben die Reste bzw. Parameter R, R² bis R¹⁶, A, Ar, Het¹, Het², Hal, k, m, n, p, t und E die bei Formel I angegebenen Bedeutungen, falls nicht ausdrücklich etwas anderes angegeben ist.

Falls eine Verbindung der Formel I mehrere Reste gleicher Bezeichnung (z.B. A, Alkenyl, Alkinyl, Ar, R⁶ , R⁷ oder Het²) enthält, können diese jeweils gleich oder voneinander verschieden sein.

In den vorstehenden Formeln hat A 1-6, vorzugsweise 1, 2, 3 oder 4 C-Atome. A bedeutet vorzugsweise Methyl, weiterhin Ethyl, Propyl, Isopropyl, Butyl, Isobutyl, sek.-Butyl, oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl. Alkenyl steht vorzugsweise für Vinyl, 1- oder 2-Propenyl, 1-Butenyl, ferner 1-Pentenyl oder 1-Hexenyl. Alkinyl steht vorzugsweise für Ethinyl, 1- oder 2-Propinyl, ferner 1-Butinyl, 1-Pentinyl oder 1-Hexinyl. Falls mehrere Reste A, Alkenyl oder Alkinyl in einer Verbindung der Formel I vorhanden sind, so können sie gleich oder voneinander verschieden sein.

Hal bedeutet vorzugsweise F, Cl oder Br, aber auch J.

R ist ein von 3H-lmidazo[4,5-c]pyridin ("3H-IP") abgeleiteter Rest, genauer 2-R³-4-oxo-5-R⁴-4,5-dihydro-3H-imidazo[4,5-c]pyridin-3-yl.

Ar ist vorzugsweise unsubstituiertes, ferner - wie angegeben - monosubstituiertes Phenyl, im einzelnen bevorzugt Phenyl, o-, m- oder p-Tolyl, o-, m- oder p-Ethylphenyl, o-, m- oder p-Trifluormethylphenyl, o-, m- oder p-Methoxyphenyl, o-, m- oder p-Ethoxyphenyl, o-, m- oder p-(Difluormethoxy)-phenyl, o-, m- oder p-(Trifluormethoxy)-phenyl, o-, m- oder p-Carboxyphenyl, o-, m- oder p-Methoxycarbonylphenyl, o-, m- oder p-Ethoxycarbonylphenyl, o-, m- oder p-Carbamoylphenyl, o-, m- oder p-(N-Methylcarbamoyl)-phenyl, o-, m- oder p-(N,N-Dimethylcarbamoyl)-phenyl, o-, m- oder p-Hydroxymethylphenyl, o-, m- oder p-Methoxymethylphenyl, o-, m- oder p-Cyanphenyl, o-, m- oder p-Nitrophenyl, o-, m- oder p-Aminophenyl, o-, m- oder p-Methylaminophenyl, o-, m- oder p-Dimethylaminophenyl, o-, m- oder p-Trifluoracetamidophenyl, o-, m- oder p-Methoxycarbonylaminophenyl, o-, m- oder p-Ethoxycarbonylaminophenyl, o-, m- oder p-Methylsulfonamidophenyl, o-, m- oder p-Trifluormethylsulfonamidophenyl, o-, m- oder p-Fluorphenyl, o-, m- oder p-Chlorphenyl, o-, m- oder p-Bromphenyl, o-, m- oder p-(1H-5-Tetrazolyl)-phenyl, ferner bevorzugt 2,3-, 2,4-, 2,5-, 2,6-, 3,4- oder 3,5-Dimethylphenyl, 2,3-, 2,4-, 2,5-, 2,6-, 3,4- oder 3,5-Dimethoxyphenyl.

Het¹ ist vorzugsweise Tetrahydro-2- oder -3-furyl, Tetrahydro-2- oder -3-thienyl, 1-, 2- oder 3-Pyrrolidinyl, 2-, 3-, 4- oder 5-Oxazolidinyl, 2-, 3-, 4- oder 5-Thiazolidinyl, 1-, 2-, 3-, 4- oder 5-Imidazolidinyl, 2-, 3- oder 4-Tetrahydropyranyl, 2-, 3- oder 4-Tetrahydrothiopyranyl, 1-, 2-, 3- oder 4-Piperidinyl, 2-, 3- oder 4-Morpholinyl, 1-, 2- oder 3-Piperazinyl, 1-Methyl-2- oder -3-pyrrolidinyl, 1-Methyl-2-, -3- oder -4-piperidinyl, 4-Methyl-2- oder -3-morpholinyl, 1-Methyl-2-, -3- oder -4-piperazinyl, 1-Phenyl-2- oder -3-pyrrolidinyl, 1-Phenyl-2-, -3- oder -4-piperidinyl, 4-Phenyl-2- oder -3-morpholinyl, 1-Phenyl-2-, -3- oder 4-piperazinyl, 2-Oxo-3-, -4- oder -5-oxazolidinyl, 2-Oxo-3-, -4- oder -5-thiazolidinyl, 2-Oxo-1-, -3-, -4- oder -5-imidazolidinyl, 2,4-Dioxo-1-, -3- oder-5-imidazolidinyl, 2-Oxo-3-phenyl-4- oder -5-oxazolidinyl, 2-Oxo-3-o-, -m- oder -p-tolyl-4- oder -5-oxazolidinyl, 2-Hydroxyimino-3-, -4- oder -5-oxazolidinyl, 2-Methoxyimino-3-, -4- oder -5-oxazolidinyl, 2-Hydroxyimino-4-oxo-3- oder -5-oxazolidinyl, 2-Methoxyimino-4-oxo-3- oder -5-oxazolidinyl.

Het² ist vorzugsweise 2- oder 3-Furyl, 2- oder 3-Thienyl, 1-, 2- oder 3-Pyrrolyl, 1-, 2-, 4- oder 5-Imidazolyl, 1-, 3-, 4- oder 5-Pyrazolyl, 2-, 4-oder 5-Oxazolyt, 3-, 4- oder 5-Isoxazolyl, 2-, 4- oder 5-Thiazolyl, 3-, 4-oder 5-Isothiazolyl, 2-, 3- oder 4-Pyridyl, 2-, 4-, 5- oder 6-Pyrimidinyl, weiterhin bevorzugt 1,2,3-Triazol-1-, -4- oder -5-yl, 1,2,4-Triazol-1-, -3-oder -5-yl, 1,2,3-Oxadiazol-4- oder -5-yl, 1,2,4-Oxadiazol-3- oder -5-yl, 1,3,4-Thiadiazol-2- oder-5-yl, 1,2,4-Thiadiazol-3- oder -4-yl, 1,2,3-Thiadiazol-4- oder -5-yl, 3- oder 4-Pyridazinyl, Pyrazinyl, 2-, 3-, 4-, 5-, 6- oder 7-Benzofuryl, 2-, 3-, 4-, 5-, 6- oder 7-Benzothienyl, 1-, 2-, 3-, 4-, 5-, 6- oder 7-Indolyl, 1-, 2-, 3-, 4-, 5-, 6- oder 7-Isoindolyl, 1-, 2-, 4- oder 5-Benzimidazolyl, 1-, 3-, 4-, 5-, 6- oder 7-Benzopyrazolyl, 2-, 4-, 5-, 6- oder 7-Benzoxazolyl, 3-, 4-, 5-, 6- oder 7-Benzisoxazolyl, 2-, 4-, 5-, 6- oder 7-Benzthiazolyl, 2-, 4-, 5-, 6- oder 7-Benzisothiazolyl, 4-, 5-, 6- oder 7-Benz-2-1,3-oxadiazolyl, 2-, 3-, 4-, 5-, 6-, 7- oder 8-Chinolinyl, 1-, 3-, 4-, 5-, 6-, 7-oder 8-Isochinolinyl, 3-, 4-, 5-, 6-, 7- oder 8-Cinnolinyl, 2-, 4-, 5-, 6-, 7-oder 8-Chinazolinyl, 1H-1-, -2-, -5-, -6- oder -7-Imidazo[4,5-b]pyridyl, 3H-2-, -3-, -5-, -6- oder -7-Imidazo[4,5-b]pyridyl, 1H-1-, -2-, -4-, -6- oder -7-Imidazo[4,5-c]pyridyl, 3H-2-, -3-, -4-, -6- oder -7-Imidazo[4,5-c]pyridyl.

In den Begriff "Het²" eingeschlossen sind auch die homologen Reste, in denen der heteroaromatische Ring durch eine oder mehrere, vorzugsweise 1 oder 2, A-Gruppen, vorzugsweise Methyl- und/oder Ethylgruppen substituiert ist, z.B. 3-, 4- oder 5-Methyl-2-furyl, 2-, 4- oder 5-Methyl-3-furyl, 2,4-Dimethyl-3-furyl, 3-, 4- oder 5-Methyl-2-thienyl, 3-Methyl-5-tert.-butyl-2-thienyl, 2-, 4- oder 5-Methyl-3-thienyl, 2- oder 3-Methyl-1-pyrrolyl, 1-, 3-, 4- oder 5-Methyl-2-pyrolyl, 3,5-Dimethyl-4-ethyl-2-pyrrolyl, 2-, 4-oder 5-Methyl-1-imidazolyl, 4-Methyl-5-pyrazolyl, 4- oder 5-Methyl-3-isoxazolyl, 3- oder 5-Methyl-4-isoxazolyl, 3- oder 4-Methyl-5-isoxazolyl, 3,4-Dimethyl-5-isoxazolyl, 4- oder 5-Methyl-2-thiazolyl, 4- oder 5-Ethyl-2-thiazolyl, 2- oder 5-Methyl-4-thiazolyl, 2- oder 4-Methyl-5-thiazolyl, 2,4-Dimethyl-5-thiazolyl, 3-, 4-, 5- oder 6-Methyl-2-pyridyl, 2-, 4-, 5- oder 6-Methyl-3-pyridyl, 2- oder 3-Methyl-4-pyridyl, 4-Methyl-2-pyrimidinyl, 4,5-Dimethyl-2-pyrimidinyl, 2-, 5- oder 6-Methyl-4-pyrimidinyl, 2,6-Dimethyl-4-pyrimidinyl, 3-, 4-, 5-, 6- oder 7-Methyl-2-benzofuryl, 2-Ethyl-3-benzofuryl, 3-, 4-, 5-, 6- oder 7-Methyl-2-benzothienyl, 3-Ethyl-2-benzothienyl, 1-, 2-, 4-, 5-, 6- oder 7-Methyl-3-indolyl, 1-Methyl-5- oder 6-benzimidazolyl, 1-Ethyl-5- oder 6-benzimidazolyl.

Die Gruppen -CₖH₂ₖ-, -CₙH₂ₙ-, -CₚH₂ₚ- und -CₙH₂ₜ- sind vorzugsweise geradkettig und stehen somit bevorzugt für -(CH₂)ₙ-, -(CH₂)ₖ-, -(CH₂)ₚ- und -(CH₂)ₜ-, insbesondere für -CH₂-, ferner für -CH₂CH₂-, -(CH₂)₃-, -(CH₂)₄-, -(CH₂)₅- oder -(CH₂)₆-, aber auch z.B. für -CH(CH₃)-, -CH₂-CH(CH₃)- oder -C(CH₃)₂-. Der Parameter k kann bevorzugt auch 0 sein, so daß die Gruppe -CₖH₂ₖ- fehlt.

Der Rest R³ ist vorzugsweise geradkettig und steht bevorzugt für A, insbesondere Ethyl, Propyl oder Butyl, ferner Methyl, Pentyl oder Hexyl, sowie für Cycloalkyl mit 3-7 C-Atomen, insbesondere Cyclopropyl, ferner Cyclobutyl, Cyclopentyl, Cyclohexyl, Cycloheptyl, weiterhin insbesondere für Alkenyl mit vorzugsweise 3-6 C-Atomen, vor allem Allyl oder 1-Propenyl, ferner 1-Butenyl, 1-Pentenyl oder 1-Hexenyl; für Alkinyl mit vorzugsweise 3-6 C-Atomen, vor allem Propargyl oder 1-Propinyl, ferner 1-Butinyl, 1-Pentinyl oder 1-Hexinyl; für Cycloalkylalkyl mit vorzugsweise 4-8 C-Atomen, vor allem Cyclopropylmethyl, 1- oder 2-Cyclopropylethyl, ferner Cyclobutylmethyl, Cyclopentylmethyl, Cyclohexylmethyl; für Alkoxy mit vorzugsweise 1-4 C-Atomen wie Methoxy, Ethoxy, Propoxy, Butoxy, Isobutoxy; für Alkoxyalkyl mit vorzugsweise 2-5 C-Atomen wie Methoxymethyl, Ethoxymethyl, Propoxymethyl, 2-Methoxyethyl, 3-Methoxypropyl, 2-Ethoxyethyl; für Alkylthio mit vorzugsweise 1-4 C-Atomen wie Methylthio, Ethylthio, Propylthio, Butylthio, Isobutylthio; für Alkylthioalkyl mit vorzugsweise 2-5 C-Atomen wie Methylthiomethyl, Ethylthiomethyl, Propylthiomethyl, 2-Methylthio-ethyl, 3-Methylthiopropyl, 2-Ethylthio-ethyl.

Der Rest R⁴ steht bevorzugt für R⁸, insbesondere CH₃, CF₃, C₂H₅, C₂F₅, CH₂CF₃, C₃H₇, CH₂CH₂CF₃, C₄H₉; Ar-C₂-C₆-alkenyl, z.B. Cinnamyl; im "Alkenyl"-Teil durch COOA substituiertes Ar-C₂-C₆-alkenyl, z.B. 3-Ethoxycarbonyl-2-phenyl-2-propen-1-yl; -CₙH₂ₙ-R⁹ (im einzelnen bevorzugt für -CH₂-R⁹), insbesondere für -CₙH₂ₙ- C₃-C₈-cycloalkyl (wie Cyclopropylmethyl), Cyclobutylmethyl, Cyclopentylmethyl, Cyclohexylmethyl, - CₙH₂ₙ-CN (wie Cyanmethyl, 2-Cyanethyl, 3-Cyanpropyl), -CₙH₂ₙ-COOA (wie Methoxycarbonylmethyl, Ethoxycarbonylmethyl, 2-Methoxycarbonylethyl, 2-Ethoxycarbonyl-ethyl), -CₙH₂ₙ-COOH (wie Carboxymethyl, 2-Carboxyethyl, 3-Carboxypropyl), -CₙH₂ₙ-Ar (wie Benzyl, 1- oder 2-Phenylethyl, 1-, 2- oder 3-Phenylpropyl, 1-, 2-, 3- oder 4-Phenylbutyl, o-, m- oder p-Fluorbenzyl, (bevorzugt) o-, m- oder p-Chlorbenzyl, o-, m- oder p-Brombenzyl, o-, m- oder p-Methylbenzyl, o-, m- oder p-Trifluormethylbenzyl, o-, m- oder p-Methoxycarbonylbenzyl, o-, m- oder p-Ethoxycarbonylbenzyl, o-, m- oder p-Cyanbenzyl, o-, m- oder p-Carboxybenzyl, o-, m- oder p-Nitrobenzyl, o-, m- oder p-Aminobenzyl, o-, m- oder p-Trifluoracetamidobenzyl, o-, m- oder p-Trifluormethylsulfonamidobenzyl, o-, m-oder p- (1H-5-Tetrazolyl)-benzyl, 2-Chlor-6-nitrobenzyl); -CₙH₂ₙ- Het¹ (bevorzugt -CH₂-Het¹ wie -CH₂-(2-oxo-3-Ar-5-oxazolidinyl), z.B. 2-Oxo-3-m-tolyl-5-oxazolidinylmethyl); -CₙH₂ₙ- Het² (bevorzugt -CH₂-Het² wie 2-oder 3-Furylmethyl, 2- oder 3-Thienylmethyl, 5-Isoxazolylmethyl, 5-Methyl-3-isoxazolylmethyl, 2-, 3- oder 4-Pyridylmethyl, Pyrazinylmethyl, 2-, 4-, 5- oder 6-Pyrimidinylmethyl, 3- oder 4-Pyridazinylmethyl, 2-, 3-, 4-, 5-, 6- oder 7-Benzofurylmethyl, 2-, 3-, 4-, 5-, 6- oder 7-Benzothienylmethyl, 2-, 3-, 4-, 5-, 6- oder 7-Indolylmethyl); -CₙH₂ₙ-(1H-5-Tetrazolyl) (wie 1H-5-Tetrazolyl-methyl, 2-(1H-5-Tetrazolyl)-ethyl, 3-(1H-5-Tetrazolyl)-propyl); -CₙH₂ₙ-CONR⁶R⁷ (worin n bevorzugt 1 oder 2, R⁶ bevorzugt H oder A und R⁷ bevorzugt H, A, Ar, ArCₙH₂ₙ oder Het² bedeuten, wie Carbamoylmethyl, 2-Carbamoylethyl, N-Methyl-carbamoylmethyl, 2-N-Methylcarbamoylethyl, N-Ethyl-carbamoylmethyl, 2-N-Ethylcarbamoyl-ethyl, N-Propyl-carbamoylmethyl, 2-N-Propyl-carbamoyl-ethyl, N-Isopropyl-carbamoylmethyl,

N-Butyl-carbamoylmethyl, 2-N-Butyl-carbamoyl-ethyl, N-Isobutyl-carbamoylmethyl, N-sek.-Butyl-carbamoylmethyl, N-tert.-Butyl-carbamoylmethyl, N,N-Dimethyl-carbamoylmethyl, 2-N,N-Dimethyl-carbamoylethyl, N-Methyl-N-ethyl-carbamoylmethyl, N,N-Diethyl-carbamoylmethyl, N,N-Dipropyl-carbamoylmethyl, N,N-Diisopropyl-carbamoylmethyl, N,N-Dibutyl-carbamoylmethyl; ferner z.B. Pyrrolidinocarbonylmethyl, Piperidinocarbonylmethyl, Morpholinocarbonylmethyl); -CₙH₂ₙ-CO- NHAr, z.B. N-Phenyl-carbamoylmethyl, 2-N-Phenyl-carbamoylethyl, N-o-, -m-oder -p-Tolyl-carbamoylmethyl, N-o-, m- oder -p-Trifluormethylphenylcarbamoylmethyl, N-o-, -m- oder -p-Carboxyphenyl-carbamoylmethyl, N-o-, -m- oder -p-Ethoxycarbonylphenyl-carbamoylmethyl, N-o-, -m- oder p-Fluorphenyl-carbamoylmethyl, N-o-, -m- oder -p-Chlorphenyl-carbamoylmethyl, N-(2,3-, N-(2,4-, N-(2,5-, N-(2,6-, N-(3,4- oder N-(3,5-Dimethylphenyl)-carbamoylmethyl, 2-N-(2,3-, 2-N-(2,4-, 2-N-(2,5-, 2-N-(2,6-, 2-N-(3,4- oder 2-N-(3,5-Dimethylphenyl)-carbamoylethyl; -CₙH₂ₙ-CO-NH-Het², z.B. N-(2-, N-(3- oder N-(4-Pyridyl)-carbamoylmethyl, 2-N-(2-Pyridyl)-carbamoylethyl, N-(2- oder N-(3-Thienyl)-carbamoylmethyl; -CₙH₂ₙ-CO-NAAr, z.B. N-Methyl-N-phenyl-carbamoylmethyl, 2-N-Methyl-N-phenyl-carbamoylethyl, N-Ethyl-N-phenyl-carbamoylmethyl; -CₙH₂ₙ-CO-NA(CₙH₂ₙ-Ar), z.B. N-Methyl-N-benzyl-carbamoylmethyl, N-Methyl-N-(2-phenylethyl)-carbamoylmethyl, N-Methyl-N-(1,1-dimethyl-2-phenylethyl)-carbamoylmethyl, 2-N-Methyl-N-(1,1-dimethyl-2-phenylethyl)-carbamoylethyl; -CₙH₂ₙ-CO-N(Ar)₂, z.B. N,N-Diphenylcarbamoylmethyl; -CₙH₂ₙ-CO-R⁸ (bevorzugt -CH₂-CO-R⁸ wie 2-Oxopropyl, 2-Oxobutyl, 3-Oxobutyl, 3-Methyl-2-oxobutyl, 3,3-Dimethyl-2-oxobutyl, 3,3,3-Trifluor-2-oxopropyl, 3,3,4,4,4-Pentafluor-2-oxobutyl);-CₙH₂ₙ-CO-Ar (bevorzugt -CH₂-CO-Ar wie Phenacyl (= 2-Oxo-2-phenyl-ethyl), o-, m-oder p-Methylphenacyl, o-, m- oder p-Ethylphenacyl, o-, m- oder p-Trifluormethylphenacyl, o-, m- oder p-Methoxyphenacyl, o-, m- oder p-Ethoxyphenacyl, o-, m- oder p-(Difluormethoxy)-phenacyl, o-, m- oder p-(Trifluormethoxy)-phenacyl, o-, m- oder p-Carboxyphenacyl, o-, m- oder p-Methoxycarbonylphenacyl, o-, m- oder p-Ethoxycarbonylphenacyl, o-, m-oder p-Cyanphenacyl, o-, m- oder p-Nitrophenacyl, o-, m- oder p-Amino-phenacyl, o-, m- oder p-Acetamidophenacyl, o-, m- oder p-Trifluoracetamidophenacyl, o-, m- oder p-Methylsulfonamidophenacyl, o-, m- oder p-Trifluormethylsulfonamidophenacyl, o-, m- oder p-(1H-5-Tetrazolyl)-phenacyl); -CₙH₂ₙ-CO-Het² (bevorzugt -CH₂-CO-Het² wie 2-Furoylmethyl, 2-Thenoylmethyl, Picolinoylmethyl, Nicotinoylmethyl, Isonicotinoylmethyl, Pyrazin-carbonylmethyl, 2-, 4-, 5- oder 6-Pyrimidincarbonylmethyl, 3- oder 4-Pyridazincarbonylmethyl, Benzofuran-2-, -3-, -4-, -5-, -6- oder -7-carbonylmethyl, Benzothiophen-2-, -3-, -4-, -5-, -6- oder -7-carbonylmethyl, Indol-2-, -3-, -4-, -5-, -6- oder -7-carbonylmethyl); -CₙH₂ₙ-CO-CH₂-NO₂, z.B. 3-Nitro-2-oxo-propyl, 4-Nitro-3-oxopropyl; -(CH₂)ₜ-CO-CₙH₂ₙ-NH-COOA, z.B. 4-BOC-amino-2-oxobutyl, 5-BOC-amino-2-oxopentyl, 6-BOC-amino-2-oxohexyl; -CₙH₂ₙ-CO-CₙH₂ₙ-NH₂, z.B. 3-Amino-2-oxo-propyl, 4-Amino-2-oxobutyl, 5-Amino-2-oxopentyl, 6-Amino-2-oxohexyl, 4-Amino-3-oxobutyl; -CₙH₂ₙ-CO-NH-SO₂Ar, z.B. N-Phenylsulfonylcarbamoylmethyl;-CₙH₂ₙ-C(=NR¹²)-A (bevorzugt -CH₂-C(=NR¹²)-A wie -CH₂-(=NOH)-CH₃,-CH₂-C(=NOCH₃)-C(CH₃)₃; -CₙH₂ₙ-S-A, z.B. Methylthiomethyl; -CₙH₂ₙ-SO-A, z.B. Methylsulfinylmethyl; -CₙH₂ₙ-SO₂-A, z.B. Methylsulfonylmethyl;-CₙH₂ₙ-S-Ar, z.B. Phenylthio-methyl; -CₙH₂ₙ-SO-Ar, z.B. Phenylsulfinyl-methyl; -CₙH₂ₙ-SO₂-Ar, z.B. Phenylsulfonyl-methyl; -CₙH₂ₙ-S-Het², z.B. (2-Thienyl)-thiomethyl; -CₙH₂ₙ-SO-Het², z.B. (2-Pyridyl)-sulfinylmethyl; -CₙH₂ₙ-SO₂-Het², z.B. (2-, (3- oder (4-Pyridyl)-sulfonylmethyl; -CH(COOA)-Ar, z.B. a-Methoxycarbonylbenzyl, α-Ethoxycarbonylbenzyl, α-Isopropoxycarbonylbenzyl; -CH(CON(A)₂)-Ar, z.B. α-(N,N-Dimethylcarbamoyl)-benzyl.

Der Rest R⁵ ist vorzugsweise A, insbesondere Methyl, Ethyl, Propyl, Butyl, Pentyl, 3-Methylbutyl, 2,2-Dimethylpropyl, Hexyl oder 4-Methylpentyl; OA, insbesondere Ethoxy, Propoxy, Methoxy, Isopropoxy, Butoxy, Isobutoxy, tert.-Butoxy, 3-Methylbutoxy; Cycloalkyl, insbesondere Cyclopropyl; Cycloalkyl-alkyl, insbesondere Cyclopropylmethyl, 2-Cyclopropyl-ethyl, Cyclopentylmethyl, 2-Cyclopentyl-ethyl, Cyclohexylmethyl, 2-Cyclohexylethyl; Ar-alkyl, insbesondere Benzyl oder 2-Phenyl-ethyl; Ar-oxyalkyl, insbesondere Phenoxymethyl; Ar-alkoxy, insbesondere Benzyloxy, 2-Phenyl-ethoxy.

Die Reste R⁶ und R⁷ stehen vorzugsweise für H oder A, R⁶ steht zusätzlich bevorzugt für Ar, Ar-CₙH₂ₙ oder Het².

Weitere bevorzugte Gruppen -NR⁶R⁷ sind diejenigen, in denen R⁶ und R⁷ zusammen eine Alkylenkette mit 2-5 C-Atomen bedeuten, die wie angegeben substituiert und/oder durch O oder durch -NR¹⁶- unterbrochen sein kann. Besonders bevorzugte Gruppen -NR⁶R⁷ dieser Art sind z.B. Aziridino, Pyrrolidino, Piperidino, Morpholino, Piperazino, 2-Oxo-pyrrolidino, 2-Alkoxycarbonyl-pyrrolidino (worin die Alkoxygruppe 1-4 C-Atome enthält) wie 2-Methoxycarbonyl-pyrrolidino oder 2-Ethoxycarbonyl-pyrrolidino, 2- oder 3-Alkanoylamino-pyrrolidino wie 2- oder 3-Acetamidopyrrolidino, 2-, 3- oder insbesondere 4-Oxo-piperidino, 2-, 3- oder insbesondere 4-Ar-piperidino wie 2-, 3- oder 4-Phenyl-piperidino, 4-o-, 4-m - oder 4-p-Methoxyphenyl-piperidino, 4-o-, 4-m- oder 4-p-Nitrophenyl-piperidino, 4-o-, 4-m- oder 4-p-Chlorphenyl-piperidino, 3-Hydroxymethyl-4-p-chlorphenyl-piperidino, 2-, 3- oder 4-(2-Thienyl)-piperidino, 2-, 3- oder 4-N,N-Dimethylcarbamoyl-piperidino, 2-, 3- oder 4-N,N-Diethylcarbamoylpiperidino, 2-, 3- oder 4-Benzoyl-piperidino, 2-, 3- oder 4-p-Methoxybenzoyl-piperidino, 4-Methylpiperazino, 4-Phenyl-piperazino, 4-o-, 4-m- oder 4-p-Methoxyphenyl-piperazino, 4-o-, 4-m- oder 4-p-Nitrophenylpiperazino, 4-o-, 4-m- oder 4-p-Chlorphenyl-piperazino, 4-(2-Pyrimidinyl)-piperazino, 4-Methoxycarbonyl-piperazino, 4-Ethoxycarbonyl-piperazino, 4-BOC-piperazino, 4-Phenylsulfonyl-piperazino, 4-p-Tolylsulfonyl-piperazino, 4-o-, 4-m- oder 4-p-Fluorphenylsulfonyl-piperazino.

Der Rest R⁸ enthält bevorzugt 1, 2 oder 3 C-Atome und bedeutet vorzugsweise Methyl, Ethyl, Trifluormethyl, Pentafluorethyl, 2,2,2-Trifluorethyl oder 3,3,3-Trifluorpropyl.

R⁹ ist vorzugsweise Ar, -COOA, -COOH oder -CO-NR⁶R⁷, femer bevorzugt -CO-R⁸, -CO-Ar, -CO-R¹⁴ oder -C(=NR¹²)-A.

R¹⁰ ist vorzugsweise COOH oder COOA.
R¹¹ ist vorzugsweise Ar, insbesondere Phenyl.
R¹² ist vorzugsweise OH oder OR¹⁶, insbesondere OA.
R¹³ ist vorzugsweise A.
R¹⁴ ist vorzugsweise -CₙH₂ₙ-NO₂ oder -CₙH₂ₙ-NR⁶R⁷, insbesondere
-CₙH₂ₙ-NH₂.
R¹⁵ ist vorzugsweise H, ferner A mit 1-4 C-Atomen.
R¹⁶ ist vorzugsweise H oder A.

Der Parameter k ist vorzugsweise 0 oder 1. Der Parameter m ist vorzugsweise 0 oder 2. Der Parameter n ist vorzugsweise 1, ferner bevorzugt 2, 3 oder 4. Der Parameter p ist vorzugsweise 1 oder 2.

Die Verbindungen der Formel I können ein oder mehrere chirale Zentren besitzen und daher in verschiedenen - optisch-aktiven oder optisch-inaktiven - Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ic bis le ausgedrückt werden, die der Formel I entsprechen und worin die nicht näher bezeichneten Reste die bei Formel I angegebenen Bedeutungen haben, worin jedoch:
- in Ic: R⁵ -CₜH₂ₜ-(C₃-C₈-Cycloalkyl) oder -CₜH₂ₜ-Ar bedeuten;
- in Id: R⁵ -CₜH₂ₜ-(C₃-C₈-Cycloalkyl) oder -CₜH₂ₜ-Ar bedeuten;
- in Ie: R⁵ -CH₂CH₂-Cyclopentyl oder -CH₂CH₂C₆H₅ bedeuten.

Unter diesen sind diejenigen Verbindungen bevorzugt, worin R³ A oder Alkenyl mit jeweils 3-6 C-Atomen oder Cyclopropyl bedeutet.

Weitere bevorzugte Gruppen von Verbindungen entsprechen der Formel I sowie den anderen vorstehend genannten Formeln, worin jedoch der Rest R⁴ folgende Bedeutungen hat:
(a) alkenyl-Ar mit 2-6 C-Atomen im "alkenyl"-Teil,
(b) -CₙH₂ₙ-R⁹,
(c) -CₙH₂ₙ-Ar,
(d) -CₙH₂ₙ-CO-NR⁶R⁷,
(e) -CH₂-CO-NR⁶R⁷, worin R⁶ und R⁷ jeweils H, A oder Phenyl bedeuten,
(f) -CH₂-CO-NR⁶R⁷, worin R⁶ und R⁷ zusammen eine Alkylenkette mit 2-5 C-Atomen bedeuten, die ein- oder mehrfach durch Carbonylsauerstoff, Ar, Het², -CO-Ar, -COOA, -CO-N(A)₂, -CH₂OH, -SO₂-Ar und/oder -NH-CO-A substituiert und/oder durch O oder durch -NR¹⁶-unterbrochen sein kann,
(g) -CH₂-CO-NR⁶R⁷, worin -NR⁶R⁷ Pyrrolidino, Piperidino oder Morpholino bedeutet,
(i) A,
(j) -CH₂Ar,
(k) -CH₂COOH,
(l) -CH₂COOA,
(m) -CH₂-CO-Ar,
(n) -CH₂-thienyl,
(o) Cinnamyl,
(p) -CH(COOA)-Ar,
(q) -CH₂-S(O)ₘ-Ar,
(r) -CH₂-S-Ar,
(s) -CH₂-SO₂Ar.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag,

Stuttgart; insbesondere aber in der EP-A2-0 430 709 und in der US PS 4 880 804) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsstoffe können, falls erwünscht, auch in situ gebildet werden, so daß man sie aus dem Reaktionsgemisch nicht isoliert, sondern sofort weiter zu den Verbindungen der Formel I umsetzt.
(a) Bevorzugt können Verbindungen der Formel I durch N-Acylierung von Verbindungen erhalten werden, die der Formel I entsprechen, aber an Stelle eines Restes R² eine -SO₂NH₂-Gruppe enthalten. Als Acylierungsmittel eignen sich z.B. Verbindungen der Formel E-CO-R⁵, worin E eine "leaving group" bedeutet, vorzugsweise Cl, Br, I oder eine reaktionsfähig funktionell abgewandelte OH-Gruppe wie Alkylsulfonyloxy mit 1-6 C-Atomen (bevorzugt Methylsulfonyloxy) oder Arylsulfonyloxy mit 6-10 C-Atomen (bevorzugt Phenyl- oder p-Tolylsulfonyloxy). Beispiele für solche Verbindungen sind Chlorameisensäure-methyl- und -ethylester; Acetylchlorid, Cyclopropancarbonylchlorid, Benzoylchlorid, Phenylacetylchlorid, 3-Phenylpropionylchlorid, Cyclopentylacetylchlorid, 3-Cyclopentylpropionylchlorid.
   Die Umsetzung wird in der Regel in Gegenwart einer oder mehrerer Base(n), vorzugsweise eines tertiären Amins, z.B. Triethylamin, Pyridin, 4-Dimethylaminopyridin vorgenommen, zweckmäßig bei Temperaturen zwischen 0 und 100°. Ein Überschuß des Amins kann auch als Lösungsmittel dienen.
(b) Weiterhin kann man eine Verbindung der Formel I durch Solvolyse (z.B. Hydrolyse) oder Hydrogenolyse aus einem ihrer funktionellen Derivate in Freiheit setzen. So kann man Carbonsäuren der Formel I, die (mindestens) eine COOH-Gruppe enthalten, erhalten durch Verseifung entsprechender Alkylester, z.B. mit NaOH oder KOH in wässeriger Lösung mit oder ohne Zusatz eines inerten organischen Lösungsmittels wie Methanol, Ethanol, THF oder Dioxan bei Temperaturen zwischen 0 und 100°, oder durch Hydrogenolyse entsprechender Benzylester, z.B. an Pd-Kohle bei Drucken zwischen 1 und 200 bar und bei Temperaturen zwischen 0 und 100° in einem der angegebenen inerten Lösungsmittel.

Die Ausgangsstoffe, insbesondere diejenigen der Formel E-CO-R⁵, sind weitgehend bekannt. Falls sie nicht bekannt sind, können sie nach bekannten Methoden in Analogie zu bekannten Stoffen hergestellt werden.

Es ist ferner möglich, eine Verbindung der Formel I in eine andere Verbindung der Formel I umzuwandeln, indem man einen oder mehrere der Reste R und/oder R² in andere Reste R und/oder R² umwandelt, z.B. indem man eine Verbindung der Formel I (R⁴ = H) mit einer Verbindung der Formel E-R⁴ (worin R⁴ von H verschieden ist) umsetzt oder Nitrogruppen (beispielsweise durch Hydrierung an Raney-Nickel oder Pd-Kohle in einem inerten Lösungsmittel wie Methanol oder Ethanol) zu Aminogruppen reduziert und/oder freie Amino- und/oder Hydroxygruppen funktionell abwandelt und/oder funktionell abgewandelte Amino- und/oder Hydroxygruppen durch Solvolyse oder Hydrogenolyse freisetzt und/oder Nitrilgruppen zu COOH-Gruppen hydrolysiert und/oder Thioethergruppen zu SO- oder SO₂-Gruppen oxydiert, z.B. mit H₂O₂ oder einer Persäure wie 3-Chlorperbenzoesäure und/oder Verbindungen der Formel I, welche eine Carbonylgruppe enthalten, in Verbindungen der Formel I umwandelt, welche eine -C(=NR¹²)-Gruppe erhalten, z.B. durch Umsetzung mit einer Verbindung der Formel H₂N-R¹² wie Ammoniak, Hydroxylamin, O-Alkyl-, O-Alkenyl-, O-Alkinyl- oder O-Aryl-hydroxylaminen, Cyanamid oder primären Aminen der Formel H₂N-R¹³, eine Carbonsäuregruppe verestert oder amidiert, z.B. durch Umsetzung mit einem Alkohol der Formel A-OH oder mit einem Amin der Formel HNR⁶R⁷ oder der Formel H₂N-CHR¹⁵-COOA.

Bei der Alkylierung von Verbindungen der Formel I (R⁴ = H) durch Reaktion mit Verbindungen der Formel E-R⁴ arbeitet man bevorzugt in einem inerten Lösungsmittel, z.B. einem Säureamid wie DMF, N-Methylpyrrolidon, 1,3-Dimethyl-2-oxohexahydropyrimidin oder Phosphorsäure-hexamethyl-triamid, einem Alkohol wie Methanol oder tert.-Butanol, einem Ether wie THF oder einem halogenierten Kohlenwasserstoff wie Dichlormethan oder Gemischen davon und/oder in Gegenwart eines Alkalimetallalkoholats wie Natriummethylat oder Kalium-tert.-butylat, eines Alkalimetallhydrids wie Natrium- oder Kaliumhydrid, eines Alkalimetallcarbonats wie Natrium- oder Kaliumcarbonat, eines Alkalimetallbicarbonats wie Natrium- oder Kaliumbicarbonat oder eines tertiären Amins wie Triethylamin oder Ethyldiisopropylamin bei Temperaturen zwischen etwa -30 und 200, vorzugsweise zwischen 20 und 60°.

Ferner kann man freie Aminogruppen in üblicher Weise mit einem Säurechlorid oder -anhydrid acylieren oder mit einem unsubstituierten oder substituierten Alkylhalogenid alkylieren, zweckmäßig in einem inerten Lösungsmittel wie Dichlormethan oder THF und/oder in Gegenwart einer Base wie Triethylamin oder Pyridin bei Temperaturen zwischen -60 und +30°.

Gewünschtenfalls kann in einer Verbindung der Formel I eine funktionell abgewandelte Amino- und/oder Hydroxygruppe durch Solvolyse oder Hydrogenolyse nach üblichen Methoden in Freiheit gesetzt werden. So kann z.B. eine Verbindung der Formel I, die eine NHCOR¹¹- oder eine COOA-Gruppe enthält, in die entsprechende Verbindung der Formel I umgewandelt werden, die stattdessen eine NH₂- oder eine HOOC-Gruppe enthält. COOA-Gruppen können z.B. mit NaOH oder KOH in Wasser, Wasser-THF oder Wasser-Dioxan bei Temperaturen zwischen 0 und 100° verseift werden.

Bei der Amidierung von Carbonsäuregruppen arbeitet man zweckmäßig nach üblichen Methoden der Peptid-Synthese, wie sie z.B. in Houben-Weyl, I.c., Band 15/II, Seiten 1-806 (1974) beschrieben sind. Die Reaktion gelingt vorzugsweise in Gegenwart eines Dehydratisierungsmittels, z.B. eines Carbodiimids wie N,N'-Dicyclohexylcarbodiimid ("DCCI"), 1,1'-Carbonyl-diimidazol oder N-3-Dimethylaminopropyl-N'-ethyl-carbodiimid ("DAPECI"), ferner Propanphosphonsäureanhydrid (vgl. Angew.Chem. 92, 129 (1980)), Diphenylphosphorylazid oder 2-Ethoxy-N-ethoxycarbonyl-1,2-dihydrochinolin, in einem inerten Lösungsmittel, z.B. einem halogenierten Kohlenwasserstoff wie Dichlormethan, einem Ether wie THF oder Dioxan, einem Amid wie DMF oder Dimethylacetamid, einem Nitril wie Acetonitril, bei Temperaturen zwischen etwa -10 und 40, vorzugsweise zwischen 0 und 30°.

An Stelle der Carbonsäuren können auch geeignete reaktionsfähige Derivate dieser Stoffe in die Reaktion eingesetzt werden, z.B. solche, in denen reaktive Gruppen intermediär durch Schutzgruppen blockiert sind. Die Säuren können z.B. in Form ihrer aktivierten Ester verwendet werden, die zweckmäßig in situ gebildet werden, z.B. durch Zusatz von 1-Hydroxybenzotriazol oder N-Hydroxysuccinimid.

Eine Base der Formel I kann mit einer Säure in das zugehörige Säureadditionssalz übergeführt werden, beispielsweise durch Umsetzung äquivalenter Mengen der Base und der Säure in einem inerten Lösungsmittel wie Ethanol und anschließendes Eindampfen. Für diese Umsetzung kommen insbesondere Säuren in Frage, die physiologisch unbedenkliche Salze liefern. So können anorganische Säuren verwendet werden, z.B. Schwefelsäure, Salpetersäure, Halogenwasserstoffsäuren wie Chlorwasserstoffsäure oder Bromwasserstoffsäure, Phosphorsäuren wie Orthophosphorsäure, Sulfaminsäure, ferner organische Säuren, insbesondere aliphatische, alicyclische, araliphatische, aromatische oder heterocyclische ein- oder mehrbasige Carbon-, Sulfon- oder Schwefelsäuen, z.B. Ameisensäure, Essigsäure, Propionsäure, Pivalinsäure, Diethylessigsäure, Malonsäure, Bernsteinsäure, Pimelinsäure, Fumarsäure, Maleinsäure, Milchsäure, Weinsäure, Äpfelsäure, Citronensäure, Gluconsäure, Ascorbinsäure, Nicotinsäure, Isonicotinsäure, Methan- oder Ethansulfonsäure, Ethandisulfonsäure, 2-Hydroxyethansulfonsäure, Benzolsulfonsäure, p-Toluolsulfonsäure, Naphthalin-mono- und disulfonsäuren, Laurylschwefelsäure. Salze mit physiologisch nicht unbedenklichen Säuren, z.B. Pikrate, können zur Isolierung und/oder Aufreinigung der Verbindungen der Formel I verwendet werden.

Andererseits können Verbindungen der Formel I, die COOH- oder z.B. Tetrazolgruppen enthalten, mit Basen (z.B. Natrium- oder Kaliumhydroxid oder -carbonat) in die entsprechenden Metall-, insbesondere Alkalimetall - oder Erdalkalimetall-, oder in die entsprechenden Ammoniumsalze umgewandelt werden. Die Kaliumsalze der Tetrazolderivate sind besonders bevorzugt.

Die Verbindungen der Formel I können ein oder mehrere chirale Zentren besitzen und dementsprechend in verschiedenen enantiomeren oder diastereomeren Formen vorliegen, die in üblicher Weise getrennt werden können. Die Formel I umschließt alle diese Formen.

Die neuen Verbindungen der Formel I und ihre physiologisch unbedenklichen Salze können zur Herstellung pharmazeutischer Präparate verwendet werden, indem man sie zusammen mit mindestens einem Träger- oder Hilfsstoff und, falls erwünscht, zusammen mit einem oder mehreren weiteren Wirkstoff(en) in eine geeignete Dosierungsform bringt. Die so erhaltenen Zubereitungen können als Arzneimittel in der Human- oder Veterinärmedizin eingesetzt werden. Als Trägersubstanzen kommen organische oder anorganische Stoffe in Frage, die sich für die enterale (z.B. orale oder rektale) oder parenterale Applikation oder für eine Applikation in Form eines Inhalations-Sprays einengen und mit den neuen Verbindungen nicht reagieren, beispielsweise Wasser, pflanzliche Öle, Benzylalkohole, Polyethylenglykole, Glycerintriacetat und andere Fettsäureglyceride, Gelatine, Sojalecithin, Kohlehydrate wie Lactose oder Stärke, Magnesiumstearat, Talk, Cellulose. Zur oralen Anwendung dienen insbesondere Tabletten, Dragees, Kapseln, Sirupe, Säfte oder Tropfen; von Interesse sind speziell Lacktabletten und Kapseln mit magensaftresistenten Überzügen bzw. Kapselhüllen. Zur rektalen Anwendung dienen Suppositorien, zur parenteralen Applikation Lösungen, vorzugsweise ölige oder wässerige Lösungen, ferner Suspensionen, Emulsionen oder Implantate. Für die Applikation als Inhalations-Spray können Sprays verwendet werden, die den Wirkstoff entweder gelöst oder suspendiert in einem Treibgasgemisch enthalten. Zweckmäßig verwendet man den Wirkstoff dabei in mikronisierter Form, wobei ein oder mehrere zusätzliche physiologisch verträgliche Lösungsmittel zugegeben sein können, z.B. Ethanol. Inhalationslösungen können mit Hilfe üblicher Inhalatoren verabfolgt werden. Die neuen Verbindungen können lyophilisiert und die erhaltenen Lyophilisate z.B. zur Herstellung von Injektionspräparaten verwendet werden. Die angegebenen Zubereitungen können sterilisiert sein und/oder Hilfsstoffe wie Konservierungs-, Stabilisierungs- und/oder Netzmittel, Emulgatoren, Salze zur Beeinflussung des osmotischen Druckes, Puffersubstanzen, Farb- und/oder Aromastoffe enthalten. Sie können, falls erwünscht, auch einen oder mehrere weitere Wirkstoffe enthalten, z.B. ein oder mehrere Vitamine, Diuretika, Antiphlogistika.

Die erfindungsgemäßen Substanzen werden in der Regel in Analogie zu anderen bekannten, im Handel befindlichen Präparaten, z.B. Captopril oder Enalapril, insbesondere aber in Analogie zu den in der US-PS 4 880 804 beschriebenen Verbindungen verabreicht, vorzugsweise in Dosierungen zwischen etwa 1 mg und 1 g, insbesondere zwischen 10 und 100 mg pro Dosierungseinheit. Die tägliche Dosierung liegt vorzugsweise zwischen etwa 0,02 und 10 mg/kg, insbesondere 0,1 und 1 mg/kg Körpergewicht. Die spezielle Dosis für jeden bestimmten Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabfolgungszeitpunkt und -weg, von der Ausscheidungsgeschwindigkeit, Arzneistoffkombination und Schwere der jeweiligen Erkrankung, welcher die Therapie gilt. Die orale Applikation ist bevorzugt.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und/oder durch Kristallisation.
IP = lmidazo[4,5-c]pyridin, IPe = lmidazo[4,5-c]pyridine.
Rf-Werte an Kieselgel; Laufmittel: Ethylacetat/Methanol 9:1;
M+ = Massenspektrum (EI)-Molpeak.

### Beispiel 1

Zu einer Lösung von 539 mg 2-Butyl-3-(2'-aminosulfonyl-3-fluorbiphenylyl-4-methyl)-4,5-dihydro-4-oxo-5-N,N-dimethyl-carbamoylmethyl-3H-IP ["A"; ölig, Rf 0,23; erhältlich durch Reaktion von 2-Butyl-4-oxo-4,5-dihydro-1(oder 3)H-IP mit 4'-Brommethyl-3-fluor-biphenyl-2-sulfonsäure-(N-tert.-butylamid) (F. 148-149°) zu 2-Butyl-3-(2'-N-tert.-butyl-aminosulfonyl-3-fluor-biphenylyl-4-methyl)-4,5-dihydro-4-oxo-3H-IP ("B"; F. 259-260°), Umsetzung mit N,N-Dimethyl-chloracetamid/K-tert.-butanolat in DMF bei 20° zu 2-Butyl-3-(2'-N-tert.-butyl-aminosulfonyl-3-fluor-biphenylyl-4-methyl)-4,5-dihydro-4-oxo-5-N,N-dimethyl-carbamoylmethyl-3H-IP (F. 177-178°) und Abspaltung der tert.-Butylgruppe mit CF₃COOH/Anisol] und 360 mg 4-Dimethylaminopyridin in 12 ml Pyridin gibt man eine Lösung von 170 mg 3-Phenylpropionylchlorid in 2 ml Pyridin, rührt das Gemisch 48 Std. bei 20°, gibt 8 ml Methanol hinzu und arbeitet wie üblich auf. Man erhält 2-Butyl-3-(2'-(3-phenylpropionylaminosulfonyl)-3-fluor-biphenylyl-4-methyl)-4,5-dihydro-4-oxo-5-N,N-dimethyl-carbamoylmethyl-3H-IP, F. 204-205°.

Analog erhält man aus "A" mit
Valerylchlorid
Hexanoylchlorid
4-Methyl-pentanoylchlorid
3,3-Dimethyl-butyrylchlorid
Heptanoylchlorid
5-Methyl-hexanoylchlorid
Cyclopropylcarbonylchlorid
3-Cyclopentyl-propionylchlorid
Phenylacetylchlorid
Ethyl-chlorformiat
Butyl-chlorformiat
Isobutyl-chlorformiat
tert.-Butyl-chlorformiat
Isopentyl-chlorformiat (= 3-Methylbutyl-chlorformiat)
Benzyl-chlorformiat
Phenoxyacetylchlorid
die nachstehenden 2-Butyl-3-(2'-R²-3-fluor-biphenylyl-4-methyl)-4,5-dihydro-4-oxo-5-N,N-dimethylcarbamoylmethyl-3H-IPe:
-2'-valeryl-aminosulfonyl-
-2'-hexanoyl-aminosulfonyl-
-2'-(4-methyl-pentanoyl-aminosulfonyl)-, F. 112-113°
-2'-(3,3-dimethyl-butyryl-aminosulfonyl)-
-2'-heptanoyl-aminosulfonyl-
-2'-(5-methyl-hexanoyl-aminosulfonyl)-
-2'-cyclopropylcarbonyl-aminosulfonyl-
-2'-(3-cyclopentyl-propionyl-aminosulfonyl)-, Rf 0,39
-2'-phenylacetyl-aminosulfonyl-
-2'-(N-ethoxycarbonyl-aminosulfonyl)-
-2'-(N-butoxycarbonyl-aminosulfonyl)-
-2'-(N-isobutoxycarbonyl-aminosulfonyl)-
-2'-(N-tert.-butoxycarbonyl-aminosulfonyl)-
-2'-(N-isopentyloxycarbonyl-aminosulfonyl)-
-2'-(N-benzyloxycarbonyl-aminosulfonyl)-
-2'-phenoxyacetyl-aminosulfonyl-.

Analog erhält man aus "B" mit den entsprechenden Halogeniden (z.B. Ethylbromid) die nachstehenden 2-Butyl-3-(2'-(N-tert.- butyl-aminosulfonyl)-3-fluor-biphenylyl-4-methyl)-4,5-dihydro-4-oxo-5-R⁴-3H-IPe:
-5-ethyl-
-5-propyl-
-5-butyl-
-5-methoxycarbonylmethyl-
-5-ethoxycarbonylmethyl-
-5-carbamoylmethyl-
-5-N-ethyl-carbamoylmethyl-
-5-N-butyl-carbamoylmethyl-
-5-N,N-diethyl-carbamoylmethyl-
-5-N-phenyl-carbamoylmethyl-
-5-(2-N-ethyl-carbamoyl-ethyl-
-5-(2-N-butyl-carbamoyl-ethyl)-
-5-(2-N,N-dimethyl-carbamoyl-ethyl)-
-5-(2-N-phenyl-carbamoyl-ethyl)-
-5-(2-oxo-propyl)-
-5-(3-oxo-butyl)-
-5-(2-oxo-3,3-dimethylbutyl)-
-5-phenacyl-
-5-(2-methoxy-phenacyl)-
-5-pyrrolidinocarbonylmethyl-
-5-piperidinocarbonylmethyl-
-5-morpholinocarbonylmethyl-
-5-benzyl-
-5-(2-methoxycarbonyl-benzyl)-
-5-(2-ethoxycarbonyl-benzyl)-
-5-(2-thienylmethyl)-
-5-(α-(N,N-dimethyl-carbamoyl)-benzyl)-
-5-(2-oxo-hexyl)-
-5-benzoylethyl-
-5-N-tert.-butyl-carbamoylmethyl-
-5-(α-isopropoxycarbonyl-benzyl)-
-5-phenylthiomethyl-
-5-phenylsulfinylmethyl-
-5-N-benzyl-carbamoylmethyl-
-5-N-propyl-carbamoylmethyl-
-5-N-(2-methylpropyl)-carbamoylmethyl-
-5-N-pentyl-carbamoylmethyl-,
daraus die nachstehenden 2-Butyl-3-(2'-(aminosulfonyl)-3-fluor-biphenylyl-4-methyl)-4,5-dihydro-4-oxo-5-R⁴-3H-IPe:
-5-ethyl-
-5-propyl-
-5-butyl-
-5-methoxycarbonylmethyl-
-5-ethoxycarbonylmethyl-
-5-carbamoylmethyl-
-5-N-ethyl-carbamoylmethyl-
-5-N-butyl-carbamoylmethyl-
-5-N,N-diethyl-carbamoylmethyl-
-5-N-phenyl-carbamoylmethyl-
-5-(2-N-ethyl-carbamoyl-ethyl)-
-5-(2-N-butyl-carbamoyl-ethyl)-
-5-(2-N,N-dimethyl-carbamoyl-ethyl)-
-5-(2-N-phenyl-carbamoyl-ethyl)-
-5-(2-oxo-propyl)-
-5-(3-oxo-butyl)-
-5-(2-oxo-3,3-dimethylbutyl)-
-5-phenacyl-
-5-(2-methoxy-phenacyl)-
-5-pyrrolidinocarbonylmethyl-
-5-piperidinocarbonylmethyl-
-5-morpholinocarbonylmethyl-
-5-benzyl-
-5-(2-methoxycarbonyl-benzyl)-
-5-(2-ethoxycarbonyl-benzyl)-
-5-(2-thienylmethyl)-
-5-(α-(N,N-dimethyl-carbamoyl)-benzyl)-
-5-(2-oxo-hexyl)-
-5-benzoylethyl-
-5-N-tert.-butylcarbamoyl-methyl-
-5-(α-isopropoxycarbonyl-benzyl)-
-5-phenylthiomethyl-
-5-phenylsulfinylmethyl-
-5-N-benzyl-carbamoylmethyl-
-5-N-propyl-carbam oylm ethyl-
-5-N-(2-methylpropyl)-carbamoylmethyl-
-5-N-pentyl-carbamoylmethyl-,
und daraus mit 3-Phenylpropionylchlorid die nachstehenden 2-Butyl-3-(2'-(3-phenylpropionylaminosulfonyl)-3-fluor-biphenylyl-4-methyl)-4,5-dihy-dro-4-oxo-5-R⁴-3H-IPe:
-5-ethyl-
-5-propyl-
-5-butyl-
-5-methoxycarbonylmethyl-, Rf (Ethylacetat) 0,44; M+ 658
-5-ethoxycarbonylmethyl-, F. 125-126°
-5-carbamoylmethyl-
-5-N-ethyl-carbamoylmethyl-
-5-N-butyl-carbamoylmethyl-, Rf 0,55; M+ 699
-5-N,N-diethyl-carbamoylmethyl-, F. 157-158°
-5-N-phenyl-carbamoylmethyl-
-5-(2-N-ethyl-carbamoyl-ethyl)-
-5-(2-N-butyl-carbamoyl-ethyl)-
-5-(2-N,N-dimethyl-carbamoyl-ethyl)-
-5-(2-N-phenyl-carbamoyl-ethyl)-
-5-(2-oxo-propyl)-, Rf 0,75; M+ 642
-5-(3-oxo-butyl)-
-5-(2-oxo-3,3-dimethylbutyl)-, F. 126-127°
-5-phenacyl-, F. 144-145°
-5-(2-methoxy-phenacyl)-
-5-pyrrolidinocarbonylmethyl-
-5-piperidinocarbonylmethyl-, F. 204-205°
-5-morpholinocarbonylmethyl-
-5-benzyl-, F. 121-122°
-5-(2-methoxycarbonyl-benzyl)-
-5-(2-ethoxycarbonyl-benzyl)-
-5-(2-thienylmethyl)-
-5-(α-(N,N-dimethyl-carbamoyl)-benzyl)-
-5-(2-oxo-hexyl)-, F. 89-90°
-5-benzoylethyl-, F. 67-68°
-5-N-tert.-butyl-carbamoyl-methyl-, F. 116-117
-5-(a-isopropoxycarbonyl-benzyl)-
-5-phenylthiomethyl-
-5-phenylsulfinylmethyl-
-5-N-benzyl-carbamoylmethyl, F. 128-129°
-5-N-propyl-carbamoylmethyl, F. 125-126°
-5-N-(2-methylpropyl)-carbamoylmethyl, F. 127-128°
-5-N-pentyl-carbamoylmethyl-.

### Beispiel 2

Analog Beispiel 1 erhält man aus 2-Ethyl-3-(2'-aminosulfonyl-3-fluor-biphenylyl-4-methyl)-4,5-dihydro-4-oxo-5-N,N-dimethylcarbamoylmethyl-3H-IP (erhältlich über 2-Ethyl-4,5-dihydro-4-oxo-1 (oder 3)H-IP, 2-Ethyl-3-(2'-N-tert.-butyl-aminosulfonyl-3-fluor-biphenylyl-4-methyl)-4,5-dihydro-4-oxo-3H-IP und 2-Ethyl-3-(2'-N-tert.-butyl-aminosulfonyl-3-fluor-biphenylyl-4-methyl)-4,5-dihydro-4-oxo-5-N,N-dimethyl-carbamoylmethyl-3H-IP) mit 3-Phenylpropionylchlorid das 2-Ethyl-3-(2'-(3-phenylpropionyl-aminosulfonyl)-3-fluor-biphenylyl-4-methyl)-4,5-dihydro-4-oxo-5-N,N-dimethyl-carbamoylmethyl-3H-IP, F. 226-227°.

Analog erhält man mit den in Beispiel 1 angegebenen Säurechloriden die nachstehenden 2-Ethyl-3-(2'-R²-3-fluor-biphenylyl-4-methyl)-4,5-dihydro-4-oxo-5-N,N-dimethyl-carbamoylmethyl-3H-IPe:
-2'-valeryl-aminosulfonyl-
-2'-hexanoyl-aminosulfonyl-
-2'-(4-methyl-pentanoyl-aminosulfonyl)-
-2'-(3,3-dimethyl-butyryl-aminosulfonyl)-
-2'-heptanoyl-aminosulfonyl-
-2'-(5-methyl-hexanoyl-aminosulfonyl)-
-2'-cyclopropylcarbonyl-aminosulfonyl-
-2'-(3-cyclopentyl-propionyl-aminosulfonyl)-
-2'-phenylacetyl-aminosulfonyl-
-2'-(N-ethoxycarbonyl-aminosulfonyl)-
-2'-(N-butoxycarbonyl-aminosulfonyl)-
-2'-(N-isobutoxycarbonyl-aminosulfonyl)-
-2'-(N-tert.-butoxycarbonyl-aminosulfonyl)-
-2'-(N-isopentyloxycarbonyl-aminosulfonyl)-
-2'-(N-benzyloxycarbonyl-aminosulfonyl)-
-2'-phenoxyacetyl-aminosulfonyl)-.

### Beispiel 3

Analog Beispiel 1 erhält man aus 2-Propyl-3-(2'-aminosulfonyl-3-fluor-biphenylyl-4-methyl)-4,5-dihydro-4-oxo-5-N,N-dimethylcarbamoylmethyl-3H-IP (erhältlich über 2-Propyl-4,5-dihydro-4-oxo-1 (oder 3)H-IP, 2-Propyl-3-(2'-N-tert.-butyl-aminosulfonyl-3-fluor-biphenylyl-4-methyl)-4,5-dihydro-4-oxo-3H-IP und 2-Propyl-3-(2'-N-tert.-butyl-aminosulfonyl-3-fluor-biphe-nylyl-4-methyl)-4,5-dihydro-4-oxo-5-N,N-dimethyl-carbamoylmethyl-3H-IP) mit 3-Phenylpropionylchlorid das 2-Propyl-3-(2'-(3-phenylpropionyl-amino-sulfonyl)-3-fluor-biphenylyl-4-methyl)-4,5-dihydro-4-oxo-5-N,N-dimethyl-carbamoylmethyl-3H-IP, F. 202-203°.

Analog erhält man mit den in Beispiel 1 angegebenen Säurechloriden die nachstehenden 2-Propyl-3-(2'-R²-3-fluor-biphenylyl-4-methyl)-4,5-dihydro-4-oxo-5-N,N-dimethyl-carbamoylmethyl-3H-IPe:
-2'-valeryl-aminosulfonyl-
-2'-hexanoyl-aminosulfonyl-
-2'-(4-methyl-pentanoyl-aminosulfonyl)-
-2'-(3,3-dimethyl-butyryl-aminosulfonyl)-
-2'-heptanoyl-aminosulfonyl-
-2'-(5-methyl-hexanoyl-aminosulfonyl)-
-2'-cyclopropylcarbonyl-aminosulfonyl-
-2'-(3-cyclopentyl-propionyl-aminosulfonyl)-
-2'-phenylacetyl-aminosulfonyl-
-2'-(N-ethoxycarbonyl-aminosulfonyl)-
-2'-(N-butoxycarbonyl-aminosulfonyl)-
-2'-(N-isobutoxycarbonyl-aminosulfonyl)-
-2'-(N-tert.-butoxycarbonyl-aminosulfonyl)-
-2'-(N-isopentyloxycarbonyl-aminosulfonyl)-
-2'-(N-benzyloxycarbonyl-aminosulfonyl)-
-2'-phenoxyacetyl-aminosulfonyl)-.

### Beispiel 4

Analog Beispiel 1 erhält man aus 2-Butyl-3-(2'-aminosulfonyl-3-fluor-biphenylyl-4-methyl)-4,5-dihydro-4-oxo-3H-IP (herstellbar aus "B" durch Abspaltung der tert.-Butylgruppe mit CF₃COOH/Anisol) und 3-Phenylpropionylchlorid das 2-Butyl-3-(2'-(3-phenylpropionyl-aminosulfonyl)-3-fluor-biphenylyl-4-methyl)-4,5-dihydro-4-oxo-3H-IP.

Analog erhält man 2-Butyl-3-(2'-(3-cyclopentyl-propionyl-aminosulfonyl)-3-fluor-biphenylyl-4-methyl)-4,5-dihydro-4-oxo-3H-IP.

### Beispiel 5

Eine Lösung von 1 g 2-Butyl-3-(2'-(3-phenylpropionyl-aminosulfonyl)-3-fluor-biphenylyl-4-methyl)-4,5-dihydro-4-oxo-5-benzyloxycarbonylmethyl-3H-IP (erhältlich durch Reaktion von "B" mit Chloressigsäurebenzylester, anschließende Abspaltung der tert.-Butylgruppe und Acylierung mit 3-Phenylpropionylchlorid) in 25 ml Methanol wird bei Normaldruck und 20° bis zum Stillstand an 0,2 g 5%ig. Pd/c hydriert. Man filtriert, dampft ein und erhält 2-Butyl-3-(2'-(3-phenylpropionyl-aminosulfonyl)-3-fluor-biphenylyl-4-methyl)-4,5-dihydro-4-oxo-5-carboxymethyl-3H-IP, F. 131-132°.

### Beispiel 6

Eine Lösung von 5,80 g 2-Butyl-3-(2'-(3-cyclopentyl-propionyl-aminosulfonyl)-3-fluor-biphenylyl-4-methyl)-4,5-dihydro-4-oxo-3H-IP (siehe Beispiel 4) in 35 ml DMF wird unter Rühren bei 20° mit 2,5 g K-tert.-butylat versetzt. Nach 45 Min. Rühren wird eine Lösung von 1,27 g Benzylchlorid in 15 ml DMF zugetropft. Man rührt noch 16 Std. bei 20°, arbeitet wie üblich auf und erhält 2-Butyl-3-(2'-(3-cyclopentyl-propionyl-aminosulfonyl)-3-fluor-biphenylyl-4-methyl)-4,5-dihydro-4-oxo-5-benzyl-3H-IP.

Analog erhält man die nachstehenden 2-Butyl-3-(2'-(3-cyclopentyl-propionyl-aminosulfonyl)-3-fluor-biphenylyl-4-methyl)-4,5-dihydro-4-oxo-5-R³-3H-IPe:
mit Ethyliodid:
-5-ethyl-
mit Bromessigsäuremethylester:
-5-methoxycarbonylmethyl-
mit Bromessigsäureethylester:
-5-ethoxycarbonylmethyl-
mit Bromessigsäure-tert.butylester:
-5-tert.butoxycarbonylmethyl-
mit Bromacetamid:
-5-carbamoylmethyl-
mit N,N-Dimethyl-chloracetamid:
-5-N,N-dimethyl-carbamoylmethyl-, Rf. 0,39
mit N,N-Diethyl-chloracetamid:
-5-N,N-diethyl-carbamoylmethyl-
mit N,N-Diphenyl-chloracetamid:
-5-N,N-diphenyl-carbamoylmethyl-
mit N-Phenyl-chloracetamid:
-5-N-phenyl-carbamoylmethyl-
mit N-Methyl-N-phenylchloracetamid:
-5-N-methyl-N-phenyl-carbamoylmethyl-
mit Bromaceton:
-5-(2-oxo-propyl)-
mit 2-Oxo-3,3-dimethyl-butylbromid:
-5-(2-oxo-3,3-dimethyl-butyl)-
mit Phenacylbromid:
-5-phenacyl-
mit 2-Methoxy-phenacylbromid:
-5-(2-methoxy-phenacyl)-
mit Bromessigsäurepyrrolidid:
-5-pyrrolidinocarbonylmethyl-
mit Bromessigsäurepiperidid:
-5-piperidinocarbonylmethyl-
mit Bromessigsäuremorpholid:
-5-morpholinocarbonylmethyl-
mit 2-Brommethyl-benzoesäureethylester:
-5-(2-ethoxycarbonyl-benzyl)-
mit 2-Chlor-benzylbromid:
-5-(2-chlorbenzyl)-
mit 2-Thienylmethylchlorid:
-5-(2-thienylmethyl)-
mit α-Brom-phenylessigsäuremethylester:
-5-(α-methoxycarbonyl-benzyl)-
mit α-Brom-phenylessigsäureisopropylester
-5-(α-isopropoxycarbonyl-benzyl)-
mit α-Brom-phenylessigsäure-N,N-dimethylamid:
-5-(α-N,N-dimethylcarbamoyl)-benzyl)-
mit Phenylthiomethylchlorid:
-5-phenylthiomethyl-
mit Phenylsulfonylmethylchlorid:
-5-phenylsulfonylmethyl-.

### Beispiel 7

Ein Gemisch von 1 g 2-Butyl-3-(2'-(3-phenylpropionyl-aminosulfonyl)-3-fluor-biphenylyl-4-methyl)-4,5-dihydro-4-oxo-5-ethoxycarbonylmethyl-3H-IP, 12 ml wässeriger 2 n NaOH-Lösung und 48 ml Methanol wird 48 Std. bei 20° gerührt, dann eingedampft. Man arbeitet mit wässeriger Salzsäure/Dichlormethan wie üblich auf und erhält 2-Butyl-3-(2'-(3-phenylpropionyl-aminosulfonyl)-3-fluor-biphenylyl-4-methyl)-4,5-dihydro-4-oxo-3H-IP-5-essigsäure.

### Beispiel 8

Analog Beispiel 3 erhält man durch Umsetzung von 2-Propyl-4,5-dihydro-4-oxo-1(oder 3)H-IP mit 4'-Brommethyl-3-fluor-biphenyl-2-sulfonsäure-(N-tert.-butylamid) 2-Propyl-3-(2'-N-tert.-butyl-aminosulfonyl-3-fluor-biphenylyl-4-methyl)-4,5-dihydro-4-oxo-3H-IP ("C"). Durch Umsetzung von "C" mit den entsprechenden Halogeniden erhält man die nachstehenden 2-Propyl-3-(2'-(N-tert.-butyl-amino-sulfonyl)-3-fluor-biphenylyl-4-methyl)-4,5-dihydro-4-oxo-5-R⁴-3H-IP-Verbindungen, worin R⁴ bedeutet:
-N-tert.-butyl-carbamoylmethyl-
-piperidino-carbonylmethyl-
-N-butyl-carbamoylmethyl-
-N-propyl-carbamoylmethyl-
-N-pentyl-carbamoylmethyl-
-N-(2-methylpropyl)-carbamoylmethyl-
-N-(3-methylbutyl)-carbamoylmethyl-
-N-(4-methylpentyl)-carbamoylmethyl-,
daraus die nachstehenden 2-Propyl-3-(2'-(aminosulfonyl)-3-fluor-biphenylyl-4-methyl)-4,5-dihydro-4-oxo-5-R⁴-3H-IP-Verbindungen, worin R⁴ bedeutet:
-N-tert.-butyl-carbamoylmethyl-
-piperidino-carbonylmethyl-
-N-butyl-carbamoylmethyl-
-N-propyl-carbamoylmethyl-
-N-pentyl-carbamoylmethyl-
-N-(2-methylpropyl)-carbamoylmethyl-
-N-(3-methylbutyl)-carbamoylmethyl-
-N-(4-methylpentyl)-carbamoylmethyl-,
und daraus mit 3-Phenylpropionylchlorid die nachstehenden 2-Propyl-3-(2'-(3-phenylpropionylaminosulfonyl)-3-fluor-biphenylyl-4-methyl)-4,5-dihydro-4-oxo-5-R⁴-3H-IP-Verbindungen:
-5-N-tert.-butyl-carbamoylmethyl-, F. 109-110°
-5-piperidino-carbamoylmethyl-, F. 206-207°
-5-N-butyl-carbamoylmethyl-, F. 121-122°
-5-N-propyl-carbamoylmethyl-, F. 167-168°
-5-N-pentyl-carbamoylmethyl-, F. 131-132°
-5-N-(2-methylpropyl)-carbamoylmethyl-, F. 124-125°
-5-N-(3-methylbutyl)-carbamoylmethyl-, F. 107-108°
-5-N-(4-methylpentyl)-carbomoylmethyl-, F. 7 5-76°.

### Beispiel 9

Analog Beispiel 1 erhält man durch Umsetzung von "B" mit N-Butyl-chloracetamid 2-Butyl-3-(2'-N-tert.-butyl-aminosulfonyl-3-fluor-biphenylyl-4-methyl)-4,5-dihydro-4-oxo-5-N-butyl-carbamoylmethyl-3H-IP ("D"). Durch Abspaltung der tert.-Butylgruppe aus "D" und Umsetzung mit 4-Methyl-pentanoylchlorid erhält man 2-Butyl-3-(2'-(4-methylpentanoyl-aminosulfonyl)-3-fluor-biphenylyl-4-methyl)-4,5-dihydro-4-oxo-5-N-butyl-carbamoylmethyl-3H-IP, M+ = 680; Rf 0,54.

### Beispiel 10

Analog Beispiel 1 erhält man durch Umsetzung von "B" mit Piperidino-N-carbonylmethylchlorid 2-Butyl-3-(2'-N-tert.-butyl-aminosulfonyl-3-fluor-biphenylyl-4-methyl)-4,5-dihydro-4-oxo-5-piperidino-carbonylmethyl-3H-IP. Daraus erhält man durch Abspaltung der tert.-Butylgruppe und Umsetzung mit Chlorkohlensäurebenzylester 2-Butyl-3-(2'-(benzyloxycarbonyl-aminosulfonyl)-3-fluor-biphenylyl-4-methyl)-4,5-dihydro-4-oxo-5-piperidinocarbonylmethyl-3H-IP, F. 173-174°.

Analog erhält man durch Umsetzung von "B" mit den entsprechenden Halogeniden (z. B. N,N-Diethyl-chloracetamid) die nachstehenden 2-Butyl-3-(2'-(N-tert.-butyl-aminosulfonyl)-3-fluor-biphenylyl-4-methyl)-4,5-dihydro-4-oxo-5-R⁴-3H-IP-Verbindungen, worin R⁴ bedeutet:
-N,N-diethyl-carbamoylmethyl-
-N,N-dimethyl-carbamoylmethyl-
-N-tert.-butyl-carbamoylmethyl-
-N-butyl-carbamoylmethyl-
-N-propyl-carbamoylmethyl-
-N-(2-methylpropyl)-carbamoylmethyl-
-N-(3-methylbutyl)-carbamoylmethyl-,
daraus die nachstehenden 2-Butyl-3-(2'-(aminosulfonyl)-3-fluor-biphenylyl-4-methyl)-4,5-dihydro-4-oxo-5-R⁴-3H-IP-Verbindungen, worin R⁴ bedeutet:
-N,N-diethyl-carbamoylmethyl-
-N,N-dimethyl-carbamoylmethyl-
-N-tert.-butyl-carbamoylmethyl-
-N-butyl-carbamoylmethyl-
-N-propyl-carbamoylmethyl-
-N-(2-methylpropyl)-carbamoylmethyl-
-N-(3-methylbutyl)-carbamoylmethyl-,
und daraus mit Chlorkohlensäurebenzylester die nachstehenden 2-Butyl-3-(2'-(benzyloxycarbonyl-aminosulfonyl)-3-fluor-biphenylyl-4-methyl)-4,5-dihydro-4-oxo-5-R⁴-3H-IP-Verbindungen, worin R⁴ bedeutet:
-N,N-diethyl-carbamoylmethyl-
-N,N-dimethyl-carbamoylmethyl-
-N-tert.-butyl-carbamoylmethyl-
-N-butyl-carbamoylmethyl-
-N-propyl-carbamoylmethyl-
-N-(2-methylpropyl)-carbamoylmethyl-
-N-(3-methylbutyl)-carbamoylmethyl-.

### Beispiel 11

Analog Beispiel 8 erhält man durch Umsetzung von "C" mit den entsprechenden Halogeniden die nachstehenden 2-Propyl-3-(2'-(N-tert.-butyl-aminosulfonyl)-3-fluor-biphenylyl-4-methyl)-4,5-dihydro-4-oxo-5-R⁴-3H-IP-Verbindungen, worin R⁴ bedeutet:
-N,N-diethyl-carbamoylmethyl-
-N,N-dimethyl-carbamoylmethyl-
-piperidino-N-carbamoylmethyl-
-N-tert.-butyl-carbamoylmethyl-
-N-butyl-carbam oylm ethyl-
-N-propyl-carbamoylmethyl-
-N-(2-methylpropyl)-carbamoylmethyl-
-N-(3-methylbutyl)-carbamoylmethyl-
daraus die nachstehenden 2-Propyl-3-(2'-(aminosulfonyl)-3-fluor-biphenylyl-4-methyl)-4,5-dihydro-4-oxo-5-R⁴-3H-IP-Verbindungen, worin R⁴ bedeutet:
-N,N-diethyl-carbamoylmethyl-
-N,N-dimethyl-carbamoylmethyl-
-piperidino-N-carbamoylmethyl-
-N-tert.-butyl-carbamoylmethyl-
-N-butyl-carbamoylmethyl-
-N-propyl-carbamoylmethyl-
-N-(2-methylpropyl)-carbamoylmethyl-
-N-(3-methylbutyl)-carbamoylmethyl-
und daraus mit Chlorkohlensäurebenzylester die nachstehenden 2-Propyl-3-(2 '-(benzyloxycarbonyl-aminosulfonyl)-3-fluor-biphenylyl-4-methyl)-4,5-dihydro-4-oxo-5-R4-3H-IP-Verbindungen, worin R⁴ bedeutet:
-N,N-diethyl-carbamoylmethyl-
-N,N-dimethyl-carbamoylmethyl-
-piperidino-N-carbamoylmethyl-
-N-tert.-butyl-carbamoylmethyl-
-N-butyl-carbamoylmethyl-
-N-propyl-carbamoylmethyl-
-N-(2-methylpropyl)-carbamoylmethyl-
-N-(3-methylbutyl)-carbamoylmethyl-.

Die nachstehenden Beispiele betreffen pharmazeutische Zubereitungen, die Wirkstoffe der Formel I oder ihre Salze enthalten.

### Beispiel A: Tabletten und Dragees

In üblicher Weise werden Tabletten folgender Zusammensetzung gepreßt, die bei Bedarf mit einer üblichen Drageedecke auf Sucrosegrundlage überzogen werden:

| | |
|---|---|
| Wirkstoff der Formel I | 100 mg |
| Mikrokristalline Cellulose | 278,8 mg |
| Lactose | 110 mg |
| Maisstärke | 11 mg |
| Magnesiumstearat | 5 mg |
| Feinteiliges Siliciumdioxid | 0,2 mg |

### Beispiel B: Hartgelatine-Kapseln

Übliche zweiteilige Hartgelatine-Kapseln werden jeweils gefüllt mit

| | |
|---|---|
| Wirkstoff der Formel I | 100 mg |
| Lactose | 150 mg |
| Cellulose | 50 mg |
| Magnesiumstearat | 6 mg |

### Beispiel C: Weichgelatine-Kapseln

Übliche Weichgelatine-Kapseln werden mit einem Gemisch aus jeweils 50 mg Wirkstoff und 250 mg Olivenöl gefüllt.

### Beispiel D: Ampullen

Eine Lösung von 200 g Wirkstoff in 2 kg 1,2-Propandiol wird mit Wasser auf 10 l aufgefüllt und in Ampullen gefüllt, so daß jede Ampulle 20 mg Wirkstoff enthält.

### Beispiel E: Wässerige Suspension für orale Applikation

Eine wässerige Suspension des Wirkstoffs wird in üblicher Weise hergestellt. Die Einheitsdosis (5 ml) enthält 100 mg Wirkstoff, 100 mg Na-Carboxymethylcellulose, 5 mg Na-Benzoat und 100 mg Sorbit.

## Patentansprüche

1. Imidazopyridinderivate der Formel I 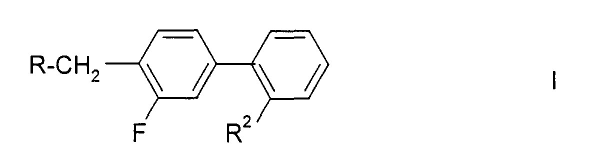 worin
R 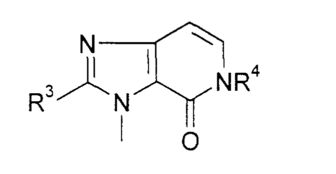
R² -SO₂NH-COR⁵,
R³ A, C₂-C₆-Alkenyl, C₂-C₆-Alkinyl, C₃-C₈-Cycloalkyl-CₖH₂ₖ-oder C₁-C₆-Alkyl, worin eine CH₂-Gruppe durch O oder S ersetzt ist,
R⁴ R⁸, unsubstituiertes oder ein- oder mehrfach durch COOH, COOA, CN, NO₂, NR⁶R⁷, NHCOR⁸, NHSO₂R⁸, Hal und/oder Ar substituiertes C₂-C₆-Alkenyl, C₂-C₆-Alkinyl, -CₙH₂ₙ-R⁹ oder -CHR¹⁰-CₖH₂ₖ-R¹¹,
R⁵ A, -CₜH₂ₜ-(C₃-C₈-Cycloalkyl), -CₜH₂ₜ-Ar, -OA, -O-CₜH₂ₜ-(C₃-C₈-Cycloalkyl), -O-CₜH₂ₜ-Ar, -CₚH₂ₚ-O-C₃-C₈-Cycloalkyl oder -CₚH₂ₚ-O-Ar,
R⁶ und R⁷ jeweils H, A, C₂-C₆-Alkenyl oder C₂-C₆-Alkinyl, Ar, ArCₙH₂ₙ- oder Het²,
R⁶ auch -CH₂COOA, -SO₂-A oder -SO₂-Ar,
R⁶ und R⁷ zusammen auch eine Alkylenkette mit 2-5 C-Atomen, die ein- oder mehrfach durch Carbonylsauerstoff, Ar, Het², -CO-Ar, -COOA, -CO-N(A)₂, -CH₂OH, -SO₂-Ar und/oder -NH-CO-A substituiert und/oder durch O oder durch -NR¹⁶- unterbrochen sein kann,
R⁸ C₁-C₅-Alkyl, worin auch ein oder mehrere H-Atom(e) durch F ersetzt sein können,
R⁹ C₃-C₈-Cycloalkyl, CN, COOA, COOH, Ar, Het¹, Het², 1H-5-Tetrazolyl, -CO-NR⁶R⁷, -CO-R⁸, -CO-Ar, -CO-Het², -CO-R¹⁴, -C(=NR¹²)-A, -C(=NR¹²)-Het², -S(O)ₘ-A, -S(O)ₘ-Ar, -S(O)ₘ-Het², -SO₂-NH-Het² oder -SO₂-OR¹⁵,
R¹⁰ COOH, COOA, CONR⁶R⁷, CN, NO₂, NHCOR¹¹, NHSO₂R¹¹ oder 1H-5-Tetrazolyl,
R¹¹ Ar oder Cycloalkyl mit 3-8 C-Atomen,
R¹² H, OH, CN, R¹³, OR¹³ oder OAr,
R¹³ A, C₂-C₆-Alkenyl oder C₂-C₆-Alkinyl,
R¹⁴ -NH-CHR¹⁵-COOH, -NH-CHR¹⁵-COOA, -CH₂S(O)ₘ-Ar, -CH₂-COOA, -CₙH₂ₙ-NO₂, -CₙH₂ₙ-NR⁶R⁷ oder -CₙH₂ₙ-NHCOOA,
R¹⁵ H oder A,
R¹⁶ H, A, Ar, COOA, Het² oder SO₂-Ar,
A C₁-C₆-Alkyl,
Ar eine unsubstituierte oder eine durch R⁸, OH, OR¹¹, COOH, COOA, CONH₂, CONHA, CON(A)₂, CH₂OH, CH₂OA, CN, NO₂, NH₂, NHA, N(A)₂, NHCOR¹¹, NHCOOA, NHSO₂R⁸, Hal und/oder 1H-5-Tetrazolyl mono- oder disubstituierte Phenylgruppe,
Het¹ einen fünf- oder sechsgliedrigen gesättigten heterocyclischen Rest mit 1 bis 3 N-, O- und/oder S-Atomen, der einfach durch Carbonylsauerstoff oder =NR¹² und/oder dessen Ring-N-Atom(e) jeweils durch A oder Ar substituiert sein kann (können),
Het² einen fünf- oder sechsgliedrigen heteroaromatischen Rest mit 1 bis 3 N-, O- und/oder S-Atomen, der auch mit einem Benzol- oder Pyridinring kondensiert sein kann,
Hal F, Cl, Br oder I,
k und t jeweils 0, 1, 2, 3 oder 4,
m 0, 1 oder 2,
n 1, 2, 3, 4, 5 oder 6 und
p 1 oder 2 bedeuten,
sowie ihre Salze.

2. 2-Butyl-3-(2'-(3-phenylpropionyl-aminosulfonyl)-3-fluor-biphenylyl-4-methyl)-4,5-dihydro-4-oxo-5-N,N-dimethyl-carbamoylmethyl-3H-imidazo[4,5-c]pyridin nach Anspruch 1.

3. Verfahren zur Herstellung von Imidazopyridinen der Formel I nach Anspruch 1 sowie ihrer Salze, dadurch gekennzeichnet, daß man
(a) eine Verbindung, die der Formel I entspricht, aber an Stelle des Restes R² eine -SO₂NH₂-Gruppe trägt, mit einer Verbindung der Formel E-COR⁵ umsetzt oder
(b) eine Verbindung der Formel I aus einem ihrer funktionellen Derivate durch Behandeln mit einem solvolysierenden oder hydrogenolysierenden Mittel in Freiheit setzt,
und/oder daß man in einer Verbindung der Formel I einen oder mehrere Rest(e) R und/oder R² in einen oder mehrere andere Reste R und/oder R² umwandelt und/oder eine Base oder Säure der Formel I in eines ihrer Salze umwandelt.

4. Verfahren zur Herstellung pharmazeutischer Zubereitungen, dadurch gekennzeichnet, daß man eine Verbindung der Formel I nach Anspruch 1 und/oder eines ihrer physiologisch unbedenklichen Säureadditionssalze zusammen mit mindestens einem festen, flüssigen oder halbflüssigen Träger- und Hilfsstoff in eine geeignete Dosierungsform bringt.

5. Pharmazeutische Zubereitung, gekennzeichnet durch einen Gehalt an mindestens einer Verbindung der Formel I nach Anspruch 1 und/oder einem ihrer physiologisch unbedenklichen Säureadditionssalze.

6. Verbindung der Formel I nach Anspruch 1 und ihre physiologisch unbedenklichen Säureadditionssalze zur Bekämpfung von Krankheiten

7. Verwendung von Verbindungen der Formel I nach Anspruch 1 und/oder ihrer physiologisch unbedenklichen Säureadditionssalze zur Herstellung eines Arzneimittels.

## Claims

1. Imidazopyridine derivatives of the formula I 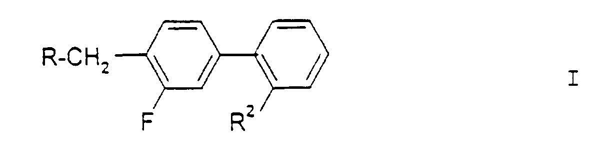 wherein
R is 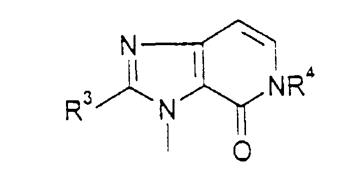
R² is -SO₂NH-COR⁵,
R³ is A, C₂-C₆-alkenyl, C₂-C₆-alkynyl, C₃-C₈-cycloalkyl-CₖH₂ₖ- or C₁-C₆-alkyl, wherein one CH₂ group is replaced by O or S,
R⁴ is R⁸, or C₂-C₆-alkenyl, C₂-C₆-alkynyl, -CₙH₂ₙ-R⁹ or -CHR¹⁰-CₖH₂ₖ-R¹¹ which is mono- or polysubstituted by COOH, COOA, CN, NO₂, NR⁶R⁷, NHCOR⁸, NHSO₂R⁸, Hal and/or Ar,
R⁵ is A, -CₜH₂ₜ- (C₃-C₈-cycloalkyl), -CₜH₂ₜ-Ar, -OA, -O-CₜH₂ₜ-(C₃-C₈-cycloalkyl), -O-CₜH₂ₜ-Ar, -CₚH₂ₚ-O-C₃-C₈-cycloalkyl or -CₚH₂ₚ-O-Ar,
R⁶ and R⁷ are each H, A, C₂-C₆-alkenyl or C₂-C₆-alkynyl, Ar, ArCₙH₂ₙ- or Het²,
R⁶ is also -CH₂COOA, -SO₂-A or -SO₂-Ar,
R⁶ and R⁷ together are also an alkylene chain having 2-5 C atoms, which can be monosubstituted or polysubstituted by carbonyl oxygen, Ar, Het², -CO-Ar, -COOA, -CO-N(A)₂, -CH₂OH, -SO₂-Ar and/or -NH-CO-A and/or interrupted by O or by -NR¹⁶-,
R⁸ is C₁-C₅-alkyl, wherein one or more H atoms can also be replaced by F,
R⁹ is C₃-C₈-cycloalkyl, CN, COOA, COOH, Ar, Het¹, Het², 1H-tetrazol-5-yl, -CC-NR⁶R⁷, -CO-R⁸, -CO-Ar, -CO-Het², -CO-R¹⁴, -C(=NR¹²)-A, -C(=NR¹²)-Het², -S(O)ₘ-A, -S(O)ₘ-Ar, -S(O)ₘ-Het², -SO₂-NH-Het² or -SO₂-OR¹⁵,
R¹⁰ is COOH, COOA, CONR⁶R⁷, CN, NO₂, NHCOR¹¹, NHSO₂R¹¹ or 1H-tetrazol-5-yl,
R¹¹ is Ar or cycloalkyl having 3-8 C atoms,
R¹² is H, OH, CN, R¹³, OR¹³ or OAr,
R¹³ is A, C₂-C₆-alkenyl or C₂-C₆-alkynyl,
R¹⁴ is -NH-CHR¹⁵-COOH, -NH-CHR¹⁵-COOA, -CH₂S(O)ₘ-Ar,-CH₂-COOA, -CₙH₂ₙ-NO₂, -CₙH₂ₙ-NR⁶R⁷ or -CₙH₂ₙ-NHCOOA,
R¹⁵ is H or A,
R¹⁶ is H, A, Ar, COOA, Het2 or SO₂-Ar,
A is C₁-C₆-alkyl,
Ar is an unsubstituted phenyl group or a phenyl group monosubstituted or disubstituted by R⁸, OH, OR¹¹, COOH, COOA, CONH₂, CONHA, CON(A)₂, CH₂OH, CH₂OA, CN, NO₂, NH₂, NHA, N(A)₂, NHCOR¹¹, NHCOOA, NHSO₂R⁸, Hal and/or 1H-tetrazol-5-yl,
Het¹ is a five- or six-membered saturated heterocyclic radical having 1 to 3 N, O and/or S atoms, which can be monosubstituted by carbonyl oxygen or =NR¹² and/or whose ring N atom(s) can in each case be substituted by A or Ar,
Het² is a five- or six-membered heteroaromatic radical having 1 to 3 N, O and/or S atoms, which can also be fused with a benzene or pyridine ring,
Hal is F, Cl, Br or I,
k and t are each 0, 1, 2, 3 or 4,
m is 0, 1 or 2,
n is 1, 2, 3, 4, 5 or 6, and
p is 1 or 2,
and their salts.

2. 2-Butyl-3-(2'-(3-phenylpropionylaminosulfonyl)-3-fluorobiphenyl-4-ylmethyl)-4,5-dihydro-4-oxo-5-N,N-di-methylcarbamoylmethyl-3H-imidazo[4,5-c]pyridine according to Claim 1.

3. Process for the preparation of imidazopyridines of the formula I according to Claim 1, and their salts, characterized in that
(a) a compound which corresponds to the formula I but carries an -SO₂NH₂- group in place of the radical R² is reacted with a compound of the formula E-COR⁵, or
(b) a compound of the formula I is freed from one of its functional derivatives by treatment with a solvolysing or hydrogenolysing agent,
and/or in that one or more radicals R and/or R² in a compound of the formula I are converted to one or more different radicals R and/or R², and/or a base or acid of the formula I is converted to one of its salts.

4. Process for the preparation of pharmaceutical formulations, characterized in that a compound of the formula I according to Claim 1, and/or one of its physiologically acceptable acid addition salts, are incorporated into a suitable dosage form together with at least one solid, liquid or semi-liquid excipient or adjunct.

5. Pharmaceutical formulation, characterized in that it contains at least one compound of the formula I according to Claim 1, and/or one of its physiologically acceptable acid addition salts.

6. Compound of the formula I according to Claim 1, and its physiologically acceptable acid addition salts, for the control of diseases.

7. Use of compounds of the formula I according to Claim 1, and/or their physiologically acceptable acid addition salts, for the preparation of a drug.

## Revendications

1. Les dérivés de l'imidazopyridine de formule I : 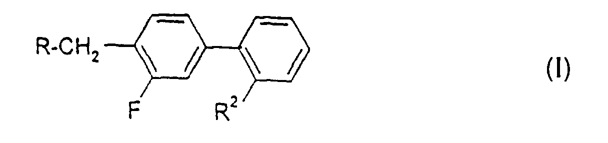 où
R représente 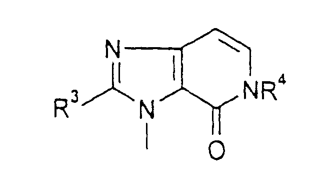
R² -SO₂NH-COR⁵,
R³ A, un alcényle en C₂-C₆, un alcynyle en C₂-C₆, un cycloalkyle en C₃-C₈-CₖH₂ₖ- ou un alkyle en C₁-C₆ où un groupe CH₂ est remplacé par O ou S,
R⁴ R⁸, un alcényle en C₂-C₆ non substitué ou mono- ou polysubstitué par COOH, COOA, CN, NO₂, NR⁶R⁷, NHCOR⁸, NHSO₂R⁸, Hal et/ou Ar, un alcynyle en C₂-C₆, -CₙH₂ₙ-R⁹ ou -CHR¹⁰-CₖH₂ₖ-R¹¹,
R⁵ A, -CₜH₂ₜ-(cycloalkyle en C₃-C₈), -CₜH₂ₜ-Ar, -OA, -O-CₜH₂ₜ-(cycloalkyle en C₃-C₈), -O-CₜH₂t-Ar, -CₚH₂ₚ-O-cycloalkyle en C₃-C₈ ou -CₚH₂ₚ-O-Ar,
R⁶ et R⁷ représentent H, A, un alcényle en C₂-C₆ ou un alcynyle en C₂-C₆, Ar, ArCₙH₂ₙ- ou Het,
R⁶ représente également -CH₂COOA, -SO₂-A ou -SO₂-Ar,
R⁶ et R⁷ représentent ensemble également une chaîne alkylène comportant 2 à 5 atomes de C, pouvant être mono- ou polysubstituée par l'oxygène du carbonyle, Ar, Het², -CO-Ar, -COOA, -CO-N(A)₂,-CH₂OH, -SO₂-Ar et/ou -NH-CO-A et/ou pouvant être interrompu par O ou par -NR¹⁶-,
R⁸ représente un alkyle en C₁-C₅ où un ou plusieurs atome(s) de H peuvent être également remplacé(s) par F,
R⁹ un cycloalkyle en C₃-C₈, CN, COOA, COOH, Ar, Het¹, Het², 1H-5-tétrazolyle, -CO-NR⁶R⁷, -CO-R⁸, -CO-Ar, -CO-Het², -CO-R¹⁴, -C(=NR¹²)-A, -C(=NR¹²)-Het², -S(O)ₘ-A, -S(O)ₘ-Ar, -S(O)ₘ-Het²,-SO₂-NH-Het² ou -SO₂-OR¹⁵,
R¹⁰ COOH, COOA, CONR⁶R⁷, CN, NO₂, NHCOR¹¹, NHSO₂R¹¹ ou 1 H-5-tétrazolyle,
R¹¹ Ar ou un cycloalkyle comportant 3 à 8 atomes de C,
R¹² H, OH, CN, R¹³, OR¹³ ou OAr,
R¹³ A, un alcényle en C₂-C₆ ou un alcynyle en C₂-C₆,
R¹⁴ -NH-CHR¹⁵-COOH, -NH-CHR¹⁵-COOA, -CH₂S(O)ₘ-Ar, -CH₂-COOA, -CₙH₂ₙ-NO₂, -CₙH₂ₙ-NR⁶R⁷ ou -CₙH₂ₙ-NHCOOA,
R¹⁵ H ou A,
R¹⁶ H, A, Ar, COOA, Het² ou SO₂-Ar,
A un alkyle en C₁-C₆,
Ar un groupe phényle non substitué ou mono- ou disubstitué par R⁸, OH, OR¹¹, COOH, COOA, CONH₂, CONHA, CON(A)₂, CH₂OH, CH₂OA, CN, NO₂, NH₂, NHA, N(A)₂, NHCOR¹¹, NHCOOA, NHSO₂R⁸, Hal et/ou 1H-5-tétrazolyle,
Het¹ un reste hétérocyclique à cinq ou six chaînons, saturé, comportant 1 à 3 atomes de N, O et/ou S pouvant être monosubstitué par l'oxygène du carbonyle ou =NR¹² et/ou le ou les atome(s) de N cyclique(s) pouvant être substitué(s) par A ou Ar,
Het² un reste hétéroaromatique à cinq ou six chaînons, comportant 1 à 3 atomes de N, O et/ou S, pouvant être également condensé avec un noyau benzénique ou pyridinique,
Hal F, Cl, Br ou 1,
k et t sont chacun égal à 0, 1, 2, 3 ou 4,
m est égal à 0, 1 ou 2,
n à 1, 2, 3, 4, 5 ou 6 et
p à 1 ou 2,
ainsi que leurs sels.

2. La 2-butyl-3-(2'-(3-phénylpropionylaminosulfonyl)-3-fluorobiphénylyl-4-méthyl)-4, 5-dihydro-4-oxo-5-N,N-diméthylcarbamoylméthyl-3H-imidazo[4, 5-c]pyridine selon la revendication 1.

3. Procédé pour la préparation des imidazopyridines de formule I selon la revendication 1, ainsi que de leurs sels, caractérisé en ce que :
(a) l'on fait réagir un composé correspondant à la formule I, mais portant à la place du reste R² un groupe -SO₂NH₂, avec un composé de formule E-COR⁵ ou
(b) l'on libère un composé de formule I de l'un de ses dérivés fonctionnels par traitement avec un agent solvolysant ou hydrogénolysant,
et/ou en ce que l'on transforme dans un composé de formule I un ou plusieurs reste(s) R et/ou R² en un ou plusieurs autre(s) reste(s) R et/ou R² et/ou en ce que l'on transforme une base ou un acide de formule I en l'un de leurs sels.

4. Procédé pour la fabrication de préparations pharmaceutiques, caractérisé en ce que l'on met sous une forme de dosage approprie, un composé de formule I selon la revendication 1 et/ou l'un de ses sels d'addition avec des acides, physiologiquement acceptables, associé à au moins un support et adjuvant solides, liquides ou semi-liquides.

5. Préparation pharmaceutique, caractérisée en ce qu'elle contient au moins un composé de formule I selon la revendication 1 et/ou l'un de ses sels d'addition avec des acides, physiologiquement acceptables.

6. Composé de formule I selon la revendication 1 et ses sels d'addition avec des acides, physiologiquement acceptables, pour lutter contre les maladies.

7. Utilisation des composés de formule I selon la revendication 1 et/ou de leurs sels d'addition avec des acides, physiologiquement acceptables, pour la fabrication d'un médicament.
